# EUROPEAN PATENT SPECIFICATION

(11) **EP 4 277 610 B1**
(45) Date of publication and mention of the grant of the patent: **26.02.2025**
(21) Application number: 22701924.7
(22) Date of filing: 18.01.2022
(51) Int. Cl.: A61K 31/05, A61K 31/365, A61P 39/00, A61P 39/06

(54) **PTEROSTILBENE AND SILIBININ FOR PREVENTING, AMELIORATING OR REDUCING RADIATION-INDUCED DISEASES**
PTEROSTILBENE UND SILIBININ ZUR VORBEUGUNG, VERBESSERUNG ODER VERMINDERUNG VON STRAHLUNGSINDUZIERTEN KRANKHEITEN
PTEROSTILBENE ET SILIBININ POUR PRÉVENIR, AMÉLIORER OU RÉDUIRE LES MALADIES INDUITES PAR LES RAYONNEMENTS

(30) Priority: 18.01.2021 EP 21382036
(43) Date of publication of application: 22.11.2023
(73) Proprietor: Universitat de València, 46010 Valencia (ES); Fundación para la Investigación del Hospital Universitario y Politécnico la Fe de la comunidad Valenciana, 46026 Valencia (ES)
(72) Inventor: ESTRELA ARIGÜEL, José María, 46010 Valencia (ES); OBRADOR PLA, María Elena, 46010 Valencia (ES); SALVADOR PALMER, María Rosario, 46010 Valencia (ES); MONTORO PASTOR, Alegría, 46026 Valencia (ES)
(74) Representative: Hoffmann Eitle
(86) International application number: PCT/EP2022/051038
(87) International publication number: WO 2022/152943

(56) References cited:
- WO-A1-2014/112194
- CN-A- 108 478 479
- JP-B2- 3 926 711
- US-A1- 2010 204 339
- US-A1- 2011 136 751
- US-A1- 2011 318 284
- US-A1- 2016 051 536
- US-A1- 2016 067 194
- J. ANTONI SIREROL ET AL: "Topical treatment with pterostilbene, a natural phytoalexin, effectively protects hairless mice against UVB radiation-induced skin damage and carcinogenesis", FREE RADICAL BIOLOGY & MEDICINE, vol. 85, 1 August 2015 (2015-08-01), US, pages 1 - 11, XP055465625, ISSN: 0891-5849, DOI: 10.1016/j.freeradbiomed.2015.03.027
- FISCHER NICOLAS ET AL: "Prevention from radiation damage by natural products", PHYTOMEDICINE, vol. 47, 2018, pages 192 - 200, XP085456771, ISSN: 0944-7113, DOI: 10.1016/J.PHYMED.2017.11.005
- JOI A NICHOLS ET AL: "Skin photoprotection by natural polyphenols: anti-inflammatory, antioxidant and DNA repair mechanisms", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 302, no. 2, 7 November 2009 (2009-11-07), pages 71 - 83, XP019780754, ISSN: 1432-069X
- MADHULIKA SINGH ET AL: "New Enlightenment of Skin Cancer Chemoprevention through Phytochemicals: In Vitro and In Vivo Studies and the Underlying Mechanisms", BIOMED RESEARCH INTERNATIONAL, vol. 2014, 1 January 2014 (2014-01-01), pages 1 - 18, XP055732673, ISSN: 2314-6133, DOI: 10.1155/2014/243452
- LIU BO ET AL: "Silibinin Alleviates the Learning and Memory Defects in Overtrained Rats Accompanying Reduced Neuronal Apoptosis and Senescence", NEUROCHEMICAL RESEARCH, SPRINGER US, NEW YORK, vol. 44, no. 8, 17 May 2019 (2019-05-17), pages 1818 - 1829, XP036844063, ISSN: 0364-3190, [retrieved on 20190517], DOI: 10.1007/S11064-019-02816-2
- HAN XIN ET AL: "Targeting Sirtuin1 to treat aging-related tissue fibrosis: From prevention to therapy", PHARMACOLOGY & THERAPEUTICS, ELSEVIER, GB, vol. 229, 2 September 2021 (2021-09-02), XP086917960, ISSN: 0163-7258, [retrieved on 20210902], DOI: 10.1016/J.PHARMTHERA.2021.107983

## Description

### TECHNICAL FIELD OF THE INVENTION

The present invention relates to the field of medicine. In particular, the present invention relates to the field of compositions and compositions for preventing, ameliorating or reducing radiation-induced diseases.

### BACKGROUND OF THE INVENTION

Nowadays, ionizing radiations are used for various medical aims. X and γ rays damage cells mainly by direct ionization of DNA and other cellular targets and by indirect effects through reactive oxygen species (ROS). The difference between γ rays and X-rays is how they are produced. Gamma rays originate from the settling process of an excited nucleus of a radionuclide after it undergoes radioactive decay whereas X-rays are produced when electrons strike a target or when electrons rearrange within an atom. X and γ rays are both types of high energy (high frequency) electromagnetic radiation. They are packets of energy (photons) that have no charge or mass. Both, X and γ rays have the same properties and health effects.

Electromagnetic radiation, including X-rays and γ-rays, damages the cells directly by ionization of DNA and other molecules, and also indirectly by the generation of reactive oxygen species. The response to radiation exposure depends on the cell type and dose of radiation, inherent tissue sensitivity and repair, and modulating intracellular factors that include cell cycle status, O₂ pressure, and levels of thiols and other antioxidants. However, the radiation protection and mitigation strategies are scarce and inefficient. Annually, millions of cancer patients undergo radiotherapy during their course of treatment and some radiological or nuclear events in recent years pose a threat to people, hence the need for radiation protection or mitigation strategies.

The development of protective agents against exposure to harmful radiations has been a subject of intense investigation for decades. Effective and safe radioprotectants are not available. To date, no drug has been developed for the treatment of high levels exposition of ionizing radiation, such as the one that causes acute hematopoietic and gastrointestinal radiation syndromes (ARS).

Radioprotectors are compounds that can reduce the direct damage caused by radiation. Radiomitigators are compounds that are able to minimize toxicity even after radiation has been delivered. In general, ideal radioprotectors or radiomitigators should be stable, with the possibility of an easy administration, with no relevant toxicity, and should protect normal tissues that are considered sensitive such that acute or late toxicities in these tissues are either dose-limiting or responsible for a significant reduction in quality of life (causing e.g. mucositis, pneumonitis, myelopathy, xerostomia, proctitis, fibrosis, or leukoencephalopathy).

A wide range of phytochemicals (compounds produced by plants, generally to help them thrive or thwart competitors, predators, or pathogens) are antioxidants and, thus, potentially radioprotective. However, their radioprotective or radiomitigator properties are usually effective during a short period of time, usually limited to a maximum of one month after radiation occurred. Therefore, new compounds and compositions that are able to treat, reduce, prevent or ameliorate the harmful effect of radiation in a subject during a continued period of times are desirable. Pterostilbene and silibinin are separately known for protecting against radiations: see document JOI A NICHOLS ET AL: "Skin photoprotection by natural polyphenols: anti-inflammatory, antioxidant and DNA repair mechanisms", ARCHIVES OF DERMATOLOGICAL RESEARCH ; FOUNDED IN 1869 AS ARCHIV FÜR DERMATOLOGIE UND SYPHILIS, SPRINGER, BERLIN, DE, vol. 302, no. 2, 7 November 2009 (2009-11-07), pages 71-83. They are also known individually for protecting against UV-induced signs of aging: see WO2014112194 and US2016067194.

The present invention provides compositions and uses thereof for the long-term protection and mitigation of diseases caused by radiation exposure.

### DESCRIPTION OF THE FIGURES

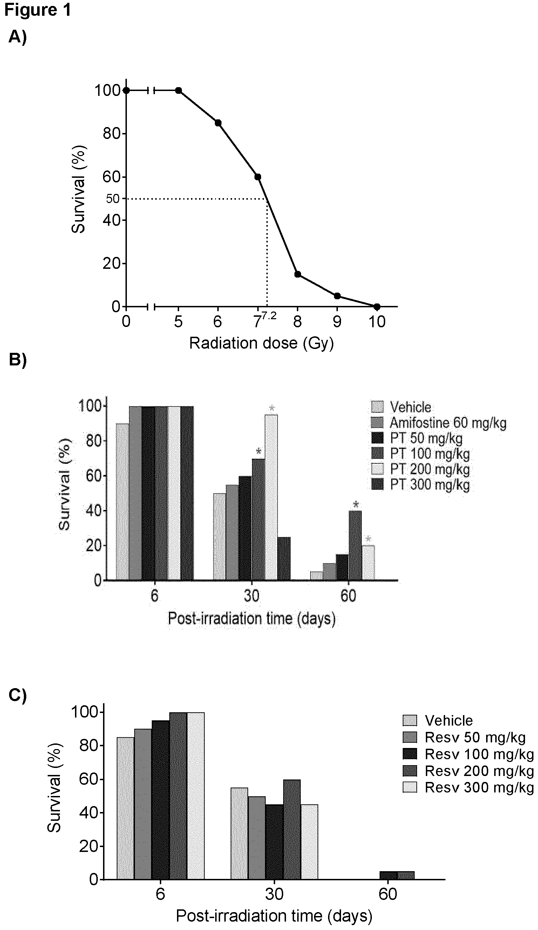

**Figure 1. A**) Radiation dose-response for a 30-day survival test in Swiss albino mice subjected to whole-body γ irradiation **B)** Sixty-days survival of mice treated with γ rays (LD50/30) and pterostilbene (PT) at different doses. Amifostine was used as a control approved radioprotector **C)** Sixty-days survival of mice treated with γ rays (LD50/30) and resveratrol (Resv) at different doses. Compounds were administered orally. In all cases, twenty mice were used per group.

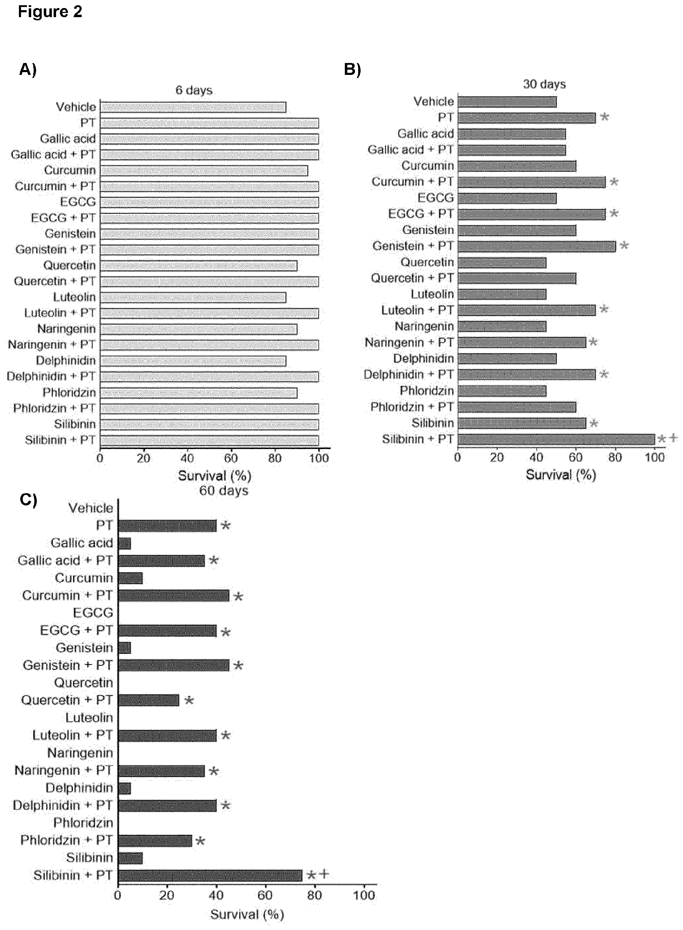

**Figure 2****.** Combination of pterostilbene (PT) and other polyphenols to prevent the lethality induced by γ radiation (LD50/30) at **A)** 6 days, **B)** 30 days and **C)** 60 days post-irradiation. Doses for each phytochemical were selected from the max non-toxic doses published. Compounds were administered orally. In all cases, twenty mice were used per group.

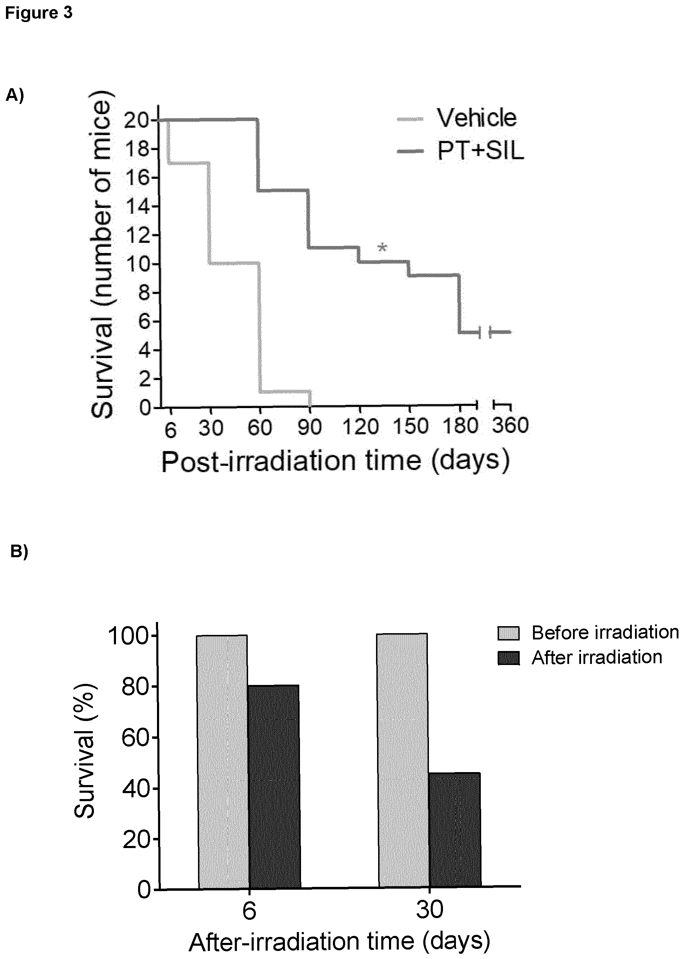

**Figure 3. A** )Effect of the combination of pterostilbene (PT) and silibinin (SIL) on the long-term survival of mice subjected to whole-body γ irradiation (LD50/30). **B)** Radioprotection elicited by the combination of PT and SIL: Comparison between administration of the polyphenols before or after the γ irradiation. Compounds were administered IP. In all cases, twenty mice were used per group.

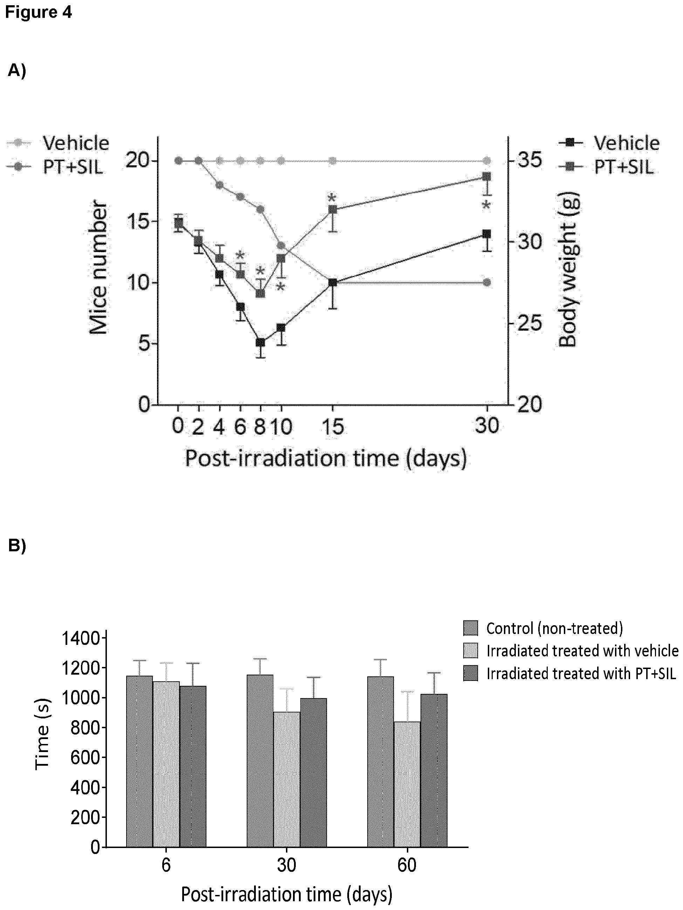

**Figure 4. A**)Effect of pterostilbene (PT) and silibinin (SIL) on the survival of γ-irradiated (LD50/30) mice and the effect on body weight. **B)** Effect of PT and SIL administration on the whole-body γ radiation-induced alteration of neuromotor functions in mice. Compounds were administered via IP. In all cases, twenty mice were used per group.

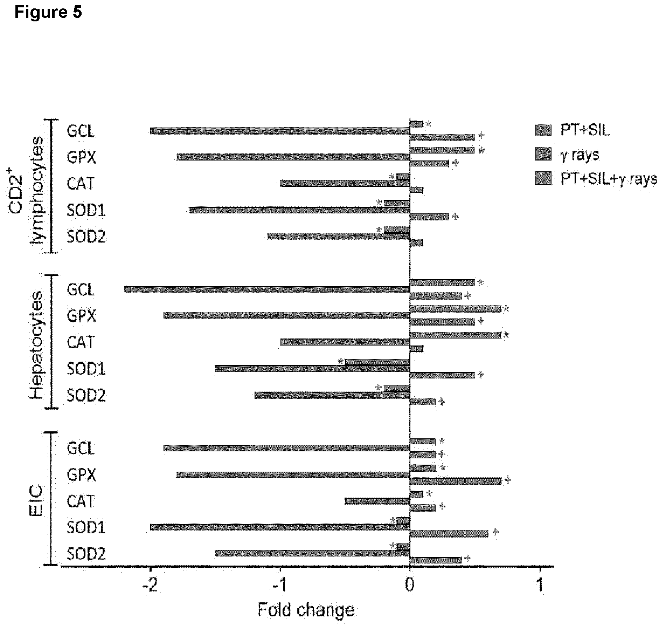

**Figure 5****.** Protective effect of pterostilbene (PT) and silibinin (SIL) on the γ radiation-induced oxidative damage expresion (RT-PCR) of different antioxidant defense-related enzyme activities in isolated cells. GCL, g-glutamyl-cysteine ligase; GPX, glutathione peroxidase; CAT, catalase; SOD, superoxide dismutase; EIC, epitelial intestinal cells. Fold change versus the expression rate of control (untreated) cells (n=5 per molecule, cell type, and treatment).

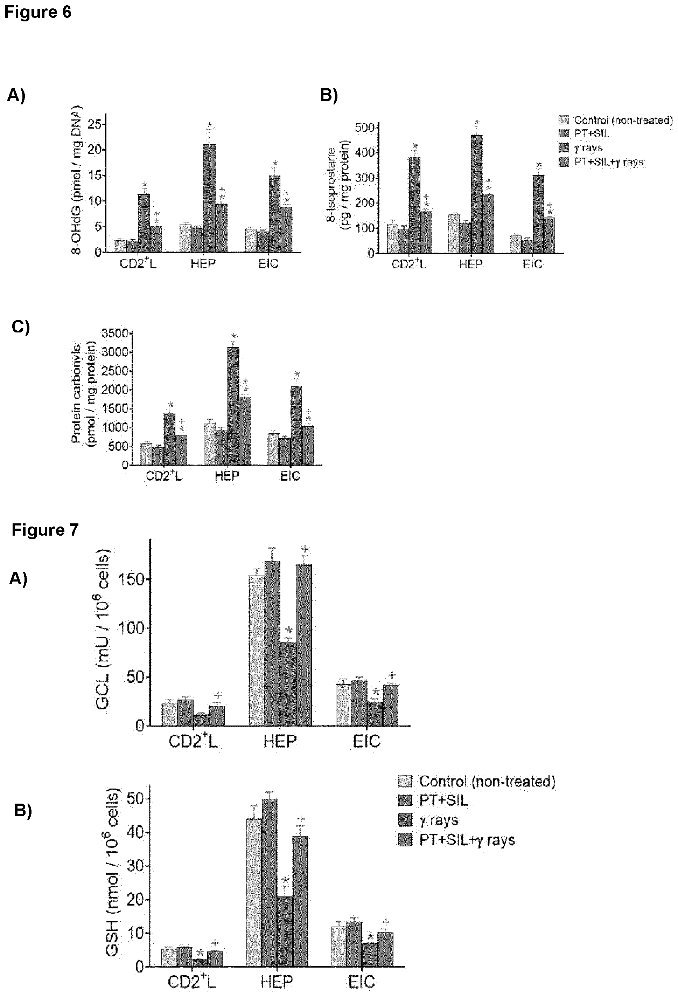

**Figure 6****.** Protective effect of pterostilbene (PT) and silibinin (SIL) on the γ radiation-induced oxidative damage in different molecular markers. **A)** 8-OHdG, **B)** 8-isoprostane and **C)** protein carbonyls (n=5 per molecule, cell type, and treatment).
**Figure 7****.** Protective effect of pterostilbene (PT) and silibinin (SIL) on the γ radiation-induced oxidative damage in the glutathione-related antioxidant defense in **A)** γ-glutamyl- cysteine ligase, **B)** glutathione (n=5 per molecule, cell type, and treatment).

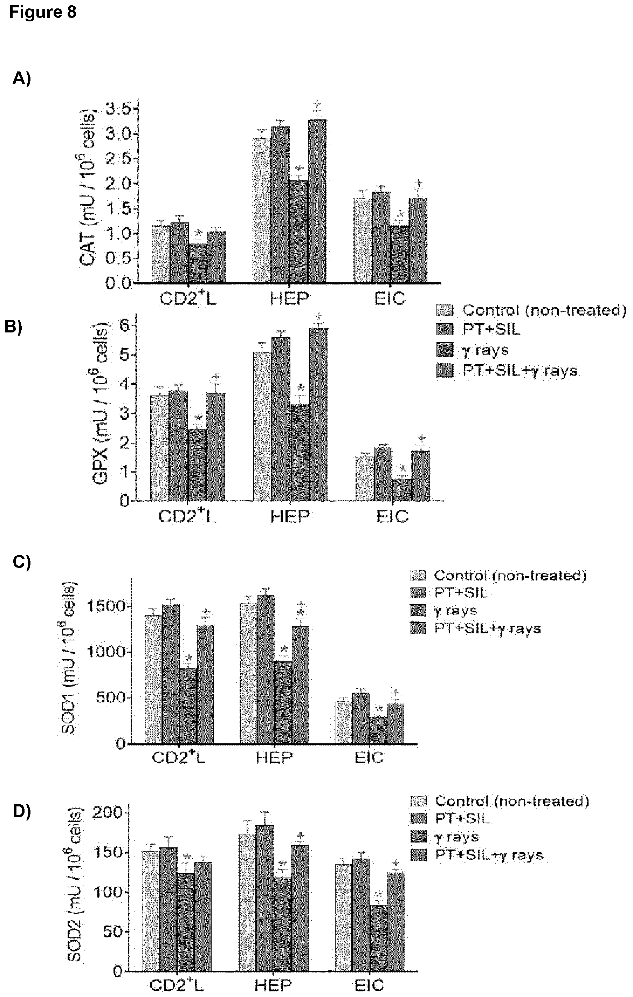

**Figure 8****.** Protective effect of pterostilbene (PT) and silibinin (SIL) on the γ radiation-induced oxidative damage in different antioxidant defense-related enzyme activities: **A)** catalase, **B)** glutathione peroxidase and **C)** and **D)** superoxide dismutases 1 and 2 (n=5 per molecule, cell type, and treatment).

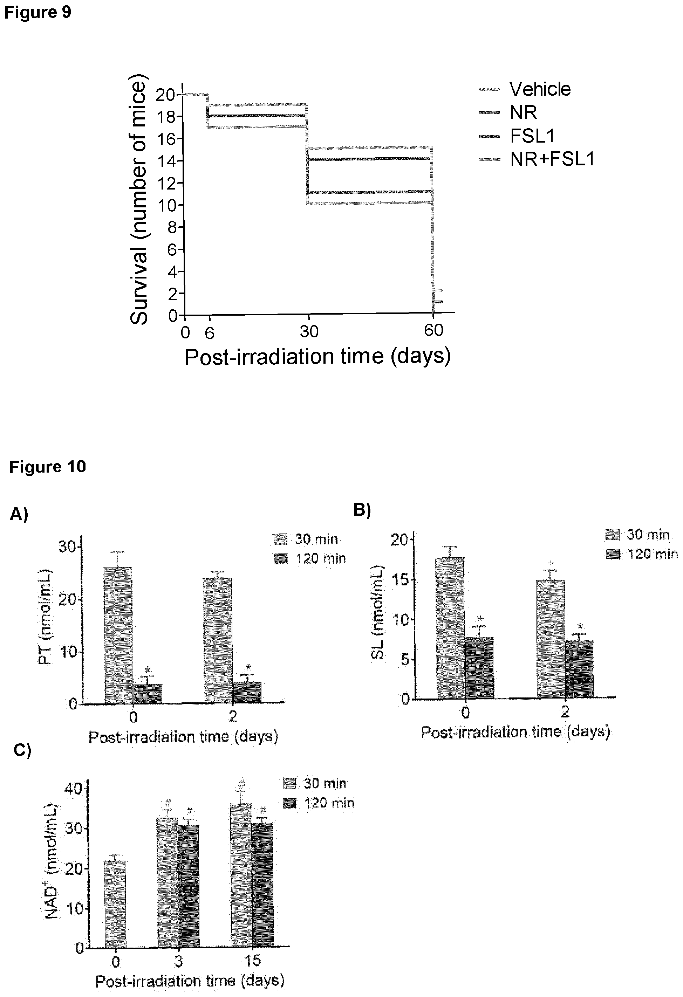

**Figure 9****.** Effect of nicotinamide riboside (NR) and fibroblast-stimulating lipopeptide (FSL-1) on the γ irradiation (LD50/30)-induced mortality. NR was administered daily for 30 days, one single dose (oral) per day, and starting right after (60 min) the irradiation. FSL-1 was administered IP, only once, 24 h after the irradiation. In all cases, twenty mice were used per group.
**Figure 10. A**)Pterostilbene (PT), **B)** Silibinin (SIL) and **C)** NAD+ levels in the circulating blood of treated and irradiated mice (6 mice per group in all cases).

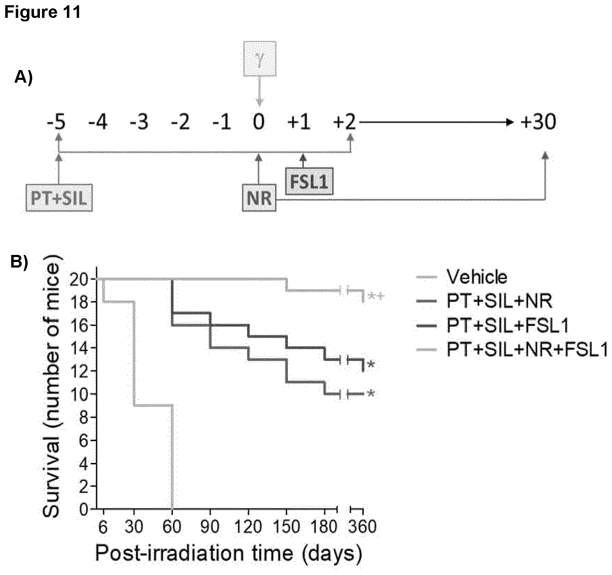

**Figure 11****.** Addition of potential radiomitigators (nicotinamide riboside, NR; and fibroblast-stimulating lipopeptide-1, FSL-1) to the radioprotective combination of pterostilbene (PT) and silibinin (SIL). **A)** administration scheme, **B)** survival versus post-irradiation time in days. In all cases, twenty mice were used per group.

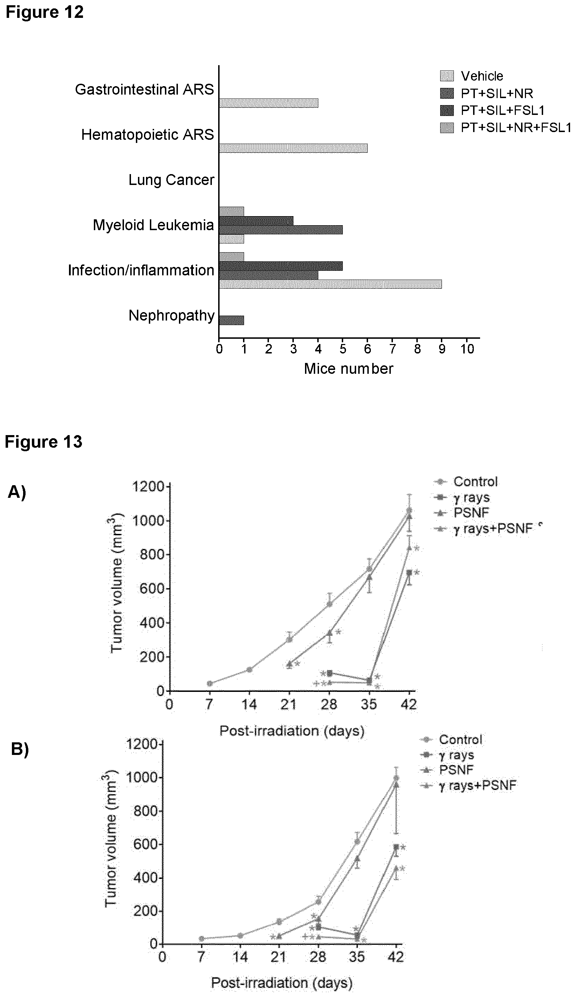

**Figure 12****.** Causes of death in γ irradiated mice (LD50/30) and the effect of the combined administration of radioprotectors and radiomitigators (PT, SIL, NR and FSL-1). In all cases, twenty mice were studied per group.
**Figure 13****.** Combined administration of the radioprotecting/radiomitigating combination PT + SIL + NR + FSL-1 (PSNF) and X rays to **A)** growing human A549 (lung adenocarcinoma) and **B)** MDA-MB-231 (triple negative mammary carcinoma) xenografts. In all cases, five mice were used per group.

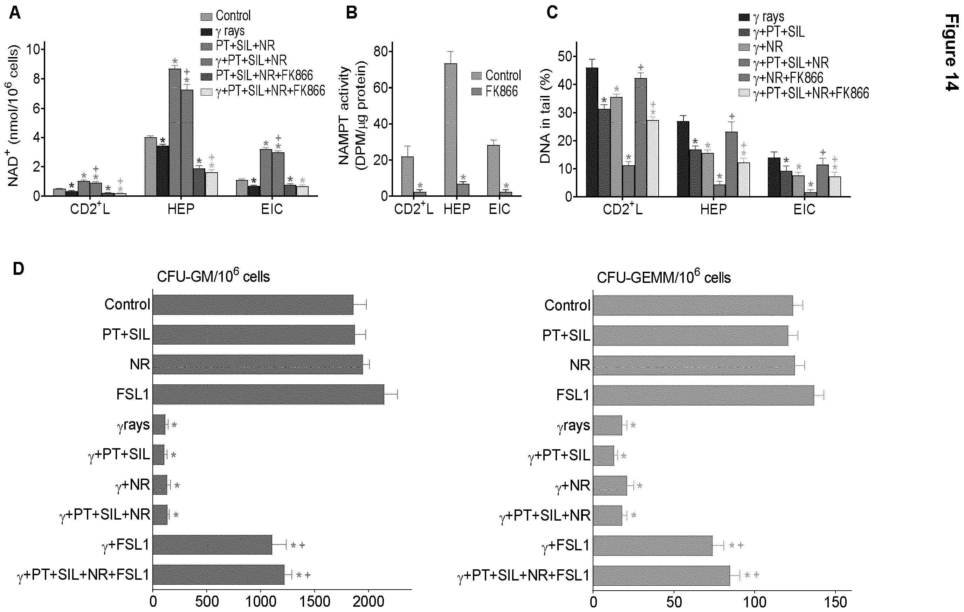

**Figure 14****.** Effect of radioprotectors and radiomitigators on cellular NAD+ content, DNA damage, and hematopoietic recovery in γ -irradiated mice. **A)** Effect of PT, SIL, NR and the specific noncompetitive inhibitor of nicotinamide phosphoribosyltransferase (NAMPT) FK866 on the NAD+ content of cells isolated from γ -irradiated mice (n = 5 per group, cell type, and treatment). FK866 was administered in vivo (15 mg/kg, twice daily by intraperitoneal injection, during 6 days and starting 3 days prior to irradiation, see Fig. 11a), and was also present in the cell cultures (100 nM). Cells were isolated 72h after irradiation. Statistical analyses were performed using Student's t-test (P<0.01; *comparing all groups vs. controls; +comparing PT+SIL+NR+ γ rays-treated mice or PT+SIL+NR+FK866+ γ rays-treated mice vs. mice treated with γ rays alone). **B)** Effect of FK866 on the cellular NAMPT activity (P<0.01; *comparing FK866-treated mice vs. controls, n = 5). Mice were treated with the inhibitor for 6 days as above (a). **C)** Effect of PT, SIL, NR and FK866 on the DNA damage in cells isolated from γ - irradiated mice (n = 5 per group, cell type, and treatment). Statistical analyses were performed using Student's t-test (P<0.01; *comparing all groups vs. γ-irradiated controls; +comparing γ-irradiated groups where FK866 was present vs. their respective controls). Mice were treated and cells isolated as above (a). **D)** Effect of PT, SIL, NR and FSL1 on the hematopoietic recovery in γ -irradiated mice. Hematopoietic colony forming cells CFU-GM (colony forming unit-granulocyte, macrophage) and CFU-GEMM (colony forming unit-granulocyte, erythroid, macrophage, megakaryocyte) were quantified in femurs isolated from control and treated mice (n = 5 per group and treatment). Mice were treated as in Fig. 11a and bone marrow cells isolated 7 days after irradiation. Statistical analyses were performed using Student's t-test (P<0.01; *comparing all groups vs. controls; +comparing the different treatments + γ irradiation vs. γ irradiation alone.

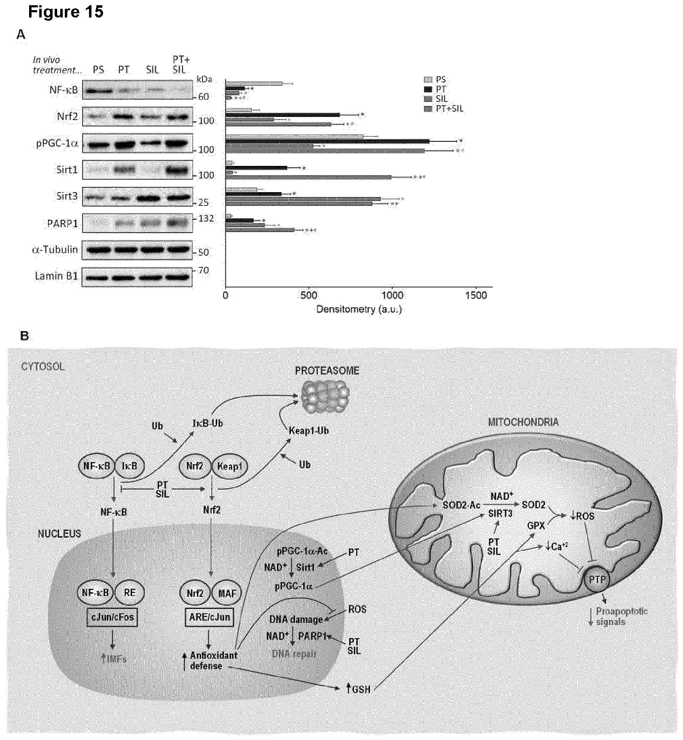

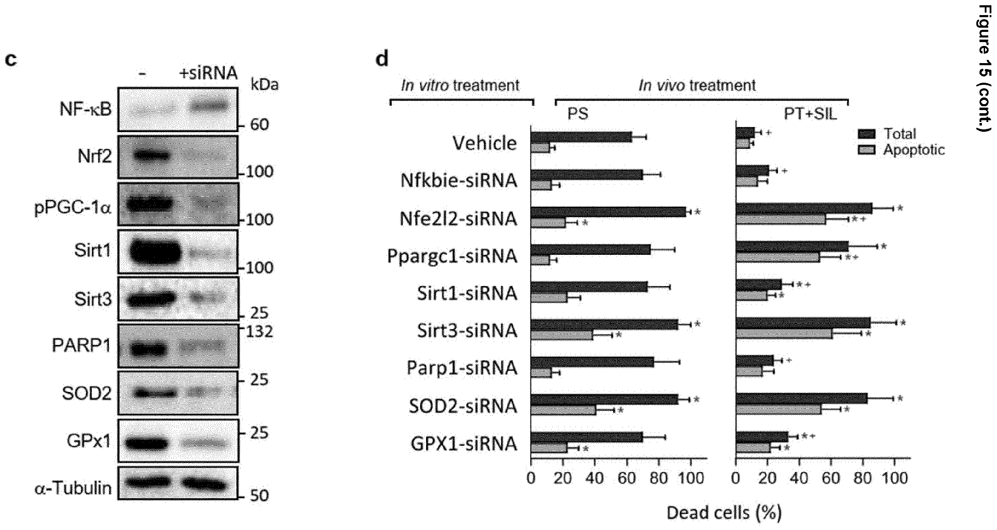

**Figure 15****.** Effect of the treatment with pterostilbene and silibinin on Nrf2-, NF-kB-, PARP1- and PGC1α-dependent mechanisms. Epithelial intestinal cells were isolated, right before the irradiation, from mice subjected to the protocol displayed in Fig. 11a. **A)** Western blots for the detection of p65NF-kB, Nrf2, PARP1 and Sirt1 in nuclear extracts, Sirt3 in mitochondrial extracts, and phosphorylated PGC-1α in total extracts. Densitometric analysis (western blots) represents the mean values ± SD for 5-6 different mice per molecule and experimental condition (P<0.01; *comparing PT-, SIL- or PT+SIL-treated mice versus physiological saline, PS-treated mice, +comparing PT+SIL- versus PT-treated mice; #comparing PT+SIL- versus SIL-treated mice). **B)** Potential molecular interactions and interrelationships between different signaling mechanisms. Ub, ubiquitin; IkB, nuclear factor of kappa light polypeptide gene enhancer in B-cells inhibitor; Keap1, Kelch-like ECH-associated protein 1; RE, response element; ARE, antioxidant response element; MAF, musculoaponeurotic fibrosarcoma oncogene homolog; Ac, acetylated; IMFs, inflammation mediating factors. **C)** Gene silencing of specific signaling-related molecules. The figure displays a representative experiment for n= 5-6 different experiments and experimental condition. PS, physiological saline. **D)** Isolated IECs were cultured for 24 h in the presence or in the absence of specific siRNAs, and thereafter were irradiated with γ rays (same dose used for the in vivo experiments). d Cell death was analyzed, as described under Methods, in isolated and cultured IEC cells 12 h after γ irradiation. PT (20 µM) and SIL (15 µM) were present in the cultures containing IECs isolated from PT+SIL-treated mice. Thereby, the effects induced by the in vivo treatment could be preserved under in vitro conditions. Data are mean values ± SD for 4-5 different experiments per molecule and experimental condition (p<0.01; *comparing, under PS or PT+SIL, treatment with a specific siRNA versus controls -treatment with vehicle-; p<0.01; +comparing data under PT+SIL versus their equivalents under PS).

### BRIEF DESCRIPTION OF THE INVENTION

In one aspect, the present invention relates to a composition comprising a) Pterostilbene, or a salt thereof, and b) Silibinin, or a salt thereof. Preferably, the composition further comprises at least one or more radiomitigator compound/s, preferably selected from a) NAD+ booster, and/or b) Fibroblast-Stimulating Lipopeptide-1, or any salts thereof.

Preferably, the composition further comprises at least two or more radiomitigators compounds, wherein the at least two or more radiomitigator compounds are a NAD+ booster and Fibroblast-Stimulating Lipopeptide-1, or any salts thereof.

Preferably, the Pterostilbene is Pterostilbene phosphate disodium salt and/or the Silibinin is silibinin-C-2',3-dihydrogen succinate, disodium salt. Preferably, the NAD+ booster is Nicotinamide Riboside.

Preferably, the composition is a pharmaceutical, nutraceutical, cosmetic or food composition.

In a further aspect, the present invention relates to a Pterostilbene, or a pharmaceutically acceptable salt thereof, for use in the prevention, amelioration or reduction of diseases induced by ionizing radiation in a subject, in combination with Silibinin, or a pharmaceutically acceptable salt thereof, wherein the administration is carried out before the radiation, or after the radiation, or during the radiation; and wherein the combination is herein understood as the simultaneous or sequential administration, in any order, of a) Pterostilbene, or a pharmaceutically acceptable salt thereof, and b) Silibinin, or a pharmaceutically acceptable salt thereof, to said subject.

Preferably, the Pterostilbene, or a pharmaceutically acceptable salt thereof, and Silibinin, or a pharmaceutically acceptable salt thereof, are administered in a sole pharmaceutical composition or in separate pharmaceutical compositions. Preferably, the administration of Pterostilbene, or a pharmaceutically acceptable salt thereof, and Silibinin, or a pharmaceutically acceptable salt thereof, is topical, oral or parenteral.

Preferably, the Pterostilbene, or a pharmaceutically acceptable salt thereof, and Silibinin, or a pharmaceutically acceptable salt thereof, are administrated several times simultaneously or sequentially before and/or after radiation, or both. Preferably, the combination is in a dosage capable of providing a radioprotective effect.

Preferably, the use further comprises the administration in combination with at least one or more radiomitigator compound/s, wherein the combination is herein understood as the simultaneous or sequential administration of a) Pterostilbene, or a pharmaceutically acceptable salt thereof, b) Silibinin, or a pharmaceutically acceptable salt thereof, and c) at least one or more radiomitigator compound/s, or any pharmaceutically acceptable salt thereof, to said subject.

Preferably, the at least one or more radiomitigator compound/s is a NAD+ booster and/or a Fibroblast-Stimulating Lipopeptide, or any pharmaceutically acceptable salts thereof.

Preferably, the use further comprises the administration in combination with at least two or more radiomitigator compounds, wherein the at least two or more radiomitigator compounds are NAD+ booster and Fibroblast-Stimulating Lipopeptide, or any pharmaceutically acceptable salts thereof. Preferably, the NAD+ booster is Nicotinamide Riboside.

Preferably, the diseases induced by ionizing radiation are related to DNA damage, radiation-induced cytotoxicity, genotoxicity and/or oxidative cellular damage, more preferably acute radiation syndrome and/or chronic radiation syndrome.

Preferably, the ionizing radiation is gamma rays or X-rays.

Preferably, the Pterostilbene is Pterostilbene phosphate disodium salt and/or the Silibinin is silibinin-C-2',3-dihydrogen succinate, disodium salt.

In a further aspect, the present invention relates to a non-therapeutic cosmetic use of Pterostilbene, or a salt thereof, in combination with Silibinin, or a salt thereof, wherein the combination is herein understood as the simultaneous or sequential administration, in any order, of a) Pterostilbene, or a salt thereof, and b) Silibinin, or a salt thereof, to said subject. Preferably, the use is to protect, ameliorate and/or reduce the adverse effects of the sun on human skin and lips, such as age spots, sunburn, sun spots, lines, fine lines, wrinkles, crow's feet, spider veins, stretch marks, dark eye circles, hyperpigmentation, hypopigmentation, discoloration, uneven skin tone, dullness, freckles, skin breakout, blemishes, skin fragility, dryness, patchiness, tactile roughness, chapping, sagginess, thinning, enlarged pores.

In a further aspect, the present invention relates to a Pterostilbene, or a salt thereof, in combination with Silibinin, or a salt thereof, for use to increase the intracellular levels of Sirtuins in a patient in need thereof, wherein said increase is as compared to the intracellular levels of Sirtuins in the same patient before treatment with Pterostilbene, or a salt thereof, in combination with Silibinin, or a salt thereof. Preferably, the patient in need thereof is a patient suffering from a neurodegenerative, cardiovascular, pulmonary, metabolic, or aged-related disorders. Preferably, the disorder is selected from the list consisting of Alzheimer or other dementias or cognitive impairment associated to aging, Parkinson, Huntington disease, Multiple sclerosis, Kennedy disease (Spinal and bulbar muscular atrophy), kidney disease, cerebrovascular disease, hypertension, Chronic obstructive pulmonary disease, Amyotrophic lateral sclerosis and Diabetes.

In a further aspect, the present invention relates to a Pterostilbene, or a salt thereof, in combination with Silibinin, or a salt thereof, for use as a nutraceutical composition, wherein the combination is herein understood as the simultaneous or sequential administration, in any order, of a) Pterostilbene, or a salt thereof, and b) Silibinin, or a salt thereof, to said subject. Preferably, the use as a nutraceutical composition is for the treatment or prevention of diseases induced by ionizing radiation, for the treatment or prevention of neurodegenerative, cardiovascular, pulmonary, metabolic, or aged-related disorders, and/or for the protection or repairment of the adverse effects of the sun on human skin or lips. Preferably, the diseases induced by ionizing radiation are related to DNA damage, radiation-induced cytotoxicity, genotoxicity and/or oxidative cellular damage. Preferably, the neurodegenerative, cardiovascular, pulmonary, metabolic, or aged-related disorder is selected from the list consisting of Alzheimer or other dementias or cognitive impairment associated to aging, Parkinson, Huntington disease, Multiple sclerosis, Kennedy disease (Spinal and bulbar muscular atrophy), kidney disease, cerebrovascular disease, hypertension, Chronic obstructive pulmonary disease, Amyotrophic lateral sclerosis and Diabetes. Preferably, the adverse effects of the sun on human skin or lips are selected from the list consisting of age spots, sunburn, sun spots, lines, fine lines, wrinkles, crow's feet, spider veins, stretch marks, dark eye circles, hyperpigmentation, hypopigmentation, discoloration, uneven skin tone, dullness, freckles, skin breakout, blemishes, skin fragility, dryness, patchiness, tactile roughness, chapping, sagginess, thinning, enlarged pores, cellulite formation, acne formation, rosacea, psoriasis, and eczema.

In a further aspect, the present invention relates to a kit of parts comprising at least two recipients comprising at least Pterostilbene, or a salt thereof, and Silibinin, or a salt thereof, and optionally at least one or more radiomitigator compound/s. Preferably, the optionally at least one or more radiomitigator compound/s is selected from the group of a) NAD+ boosters, and/or b) Fibroblast-Stimulating Lipopeptide, or any pharmaceutically acceptable salts thereof. Preferably, the NAD+ booster is Nicotinamide Riboside. Preferably, the Pterostilbene is Pterostilbene phosphate disodium salt and/or the Silibinin is silibinin-C-2',3-dihydrogen succinate, disodium salt.

### DESCRIPTION OF THE INVENTION

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series.

The term "about" when referred to a given amount or quantity is meant to include deviations of plus or minus five percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of"" does not exclude materials or steps that do not materially affect the basic and novel characteristics of the claim.

The term "disease" as used herein is intended to be generally synonymous, and is used interchangeably with, the terms "pathology", "disorder" and "condition" (as in medical condition), in that all reflect an abnormal condition of the body or of one of its parts that impairs normal functioning and is typically manifested by distinguishing signs and symptoms.

A "therapeutically effective amount" as referred herein is defined as an amount of a compound that, when administered to a subject, is sufficient to effect such treatment of a conditioned state. The "therapeutically effective amount" may vary depending on the compound, and condition being treated, the age and relative health of the subject, the route and form of administration, the judgment of the one having ordinary skill in the art, and other factors.

The term "treatment" as used herein refers to the medical care given to a patient for a disease or injury. This term includes curing the disease but also ameliorating, mitigating, or reducing the symptoms of said disease. Thus, the term "therapeutic treatment" or "treatment" as used herein refers to bringing a body from a pathological state or disease back to its normal, healthy state. The term "therapeutic treatment" or "treatment" as used herein, also refers to the mitigation, amelioration, or reduction of the harmful effect of radiation in a subject, or in some manner reducing the symptoms associated with such effects. The term "prophylactic treatment" as used herein refers to preventing a pathological state.

With "prevention" is meant keeping the disease from happening. With "amelioration" is meant an improvement in a patient's disease, or the activity of making an effort to correct, or at least make more acceptable said disease. With "reduction" or "mitigation" is meant decreasing the severity, seriousness or painfulness of the disease.

The term "subject" as used herein refers to a mammalian subject. Preferably, it is selected from a human, companion animal, non-domestic livestock or zoo animal. For example, the subject may be selected from a human, dog, cat, cow, pig, sheep, horse, bear, and so on. In a preferred embodiment, said mammalian subject is a human subject.

With "ionizing radiation" is meant the radiation consisting of particles, X-rays, or gamma rays with sufficient energy to cause ionization in the medium through which it passes. With "γ rays" or "γ radiation" is meant a penetrating form of electromagnetic radiation arising from the radioactive decay of atomic nuclei. It consists of the shortest wavelength electromagnetic waves and so imparts the highest photon energy. With "X-rays" or X radiation is meant or X-radiation, is a penetrating form of high-energy electromagnetic radiation. Most X-rays have a wavelength ranging from 10 picometers to 10 nanometers, corresponding to frequencies in the range 30 petahertz to 30 exahertz and energies in the range 124 eV to 124 keV.

With "Pterostilbene" or PT is meant 3,5-dimethoxy-4'-hydroxystilbene or a salt, solvate, isomer (enantiomers, diastereomers), hydrate, or any known derivatives, synthetic variants and acceptable formulations (such as pharmaceutical, cosmetical, nutraceutical) thereof. Pterostilbene (PT) is a natural antioxidant and a natural analogue of Resveratrol, but almost 60-100 times more potent antifungal agent that shows similar anticarcinogenic properties. It is present predominantly in blueberries and red grapes, although quantitative studies show that for every 10 parts of t-Resveratrol, there are only 1-2 parts of t-Pterostilbene. Pterostilbene corresponds to the formula:

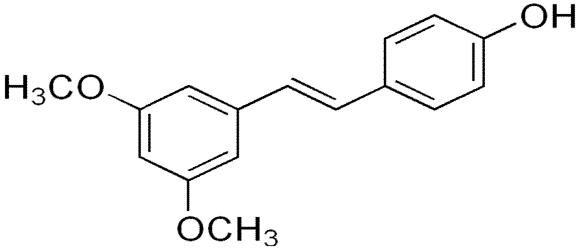

With "Silibinin" or SIL also known as silybin or silymarin (both from Silybum (milk thistle) is a flavonoid obtain from a genus of two species of thistles in the daisy family. Silibinin includes any salt, solvate, enantiomer, diastereomer, hydrate, or any known synthetic variants and acceptable formulations (such as pharmaceutical, cosmetical, nutraceutical) thereof. The plants are native to the Mediterranean regions of Europe, North Africa, and the Middle East. Silibinin (SIL), is the major active constituent of silymarin, a standardized extract of the milk thistle seeds, containing a mixture of flavonolignans consisting of silibinin, isosilibinin, silichristin, silidianin, and others. Silibinin itself is a mixture of two diastereomers, silybin A and silybin B, in approximately equimolar ratio. SIL is also known as 3,5,7-trihydroxy-2-[3-(4-hydroxy-3-methoxyphenyl)-2-(hydroxymethyl)-2,3-dihydro-1,4-benzodioxin-6-yl]-2,3-dihydrochromen-4-one with the following formula:

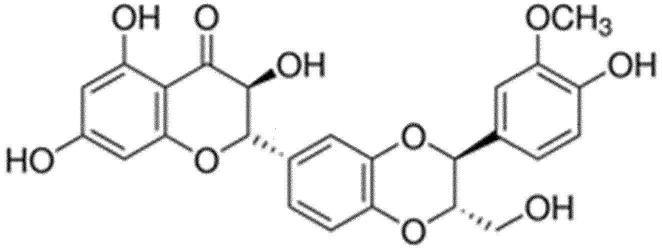

By Nicotinamide adenine dinucleotide booster, NAD+ precursors or NAD+ boosters is meant any compound as clearly disclosed on the document by Rajman et al., Cell Metab. 2018 March 06; 27(3): 529-547. doi: 1 0.1 016/j.cmet.2018.02.011, such as NAD precursors, activators of NAD synthesis, or inhibitors of NAD+ degradation. Nicotinamide includes any a salt, solvate, isomer (enantiomers, diastereomers), hydrate, or any known derivatives, synthetic variants and acceptable formulations (such as pharmaceutical, cosmetical, nutraceutical) thereof.

By "Fibroblast-Stimulating Lipopeptide" or FSL-1 is meant the Toll-Like Receptor 2/6 Agonist, or salt, solvate, enantiomer, diastereomer, hydrate, or any known synthetic variants and acceptable formulations (such as pharmaceutical, cosmetical, nutraceutical) thereof, and corresponds to the formula:

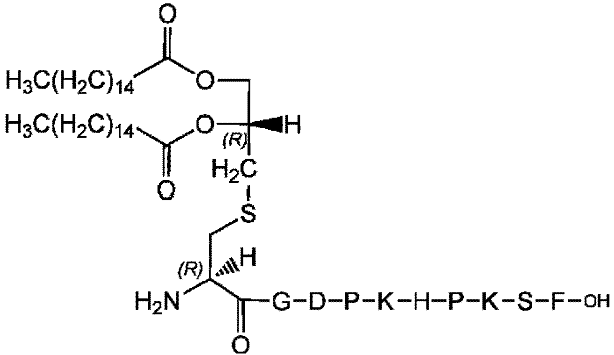

By Nicorinamide riboside is meant a pyridine-nucleoside similar to vitamin B3, functioning as a precursor to nicotinamide adenine dinucleotide or NAD+. The formula of NR is:

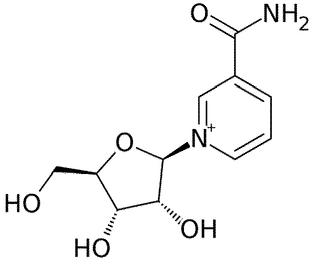

The term "kit of parts" as used herein refers to a combined preparation wherein the active ingredients are physically separated for use in a combined therapy by simultaneous administration or sequential administration to the patient.

### DETAILED DESCRIPTION

Several natural compounds are known to have radioprotective effects when administered to a subject before radiation. As shown in Figure 2A, compounds such as pterostilbene (PT), gallic acid, EGCG, etc. provide around 100% survival 6 days after radiation in Swiss albino mice. However, some compounds fail to protect the mice for longer periods of time. For instance, after 30 days (Figure 2B), the percentage of survival of Luteolin was reduced in about 40%, and after 60 days (Figure 2C), its percentage of survival was 0%. Thus, compounds able to exert high level of protection during prolonged period of times are desirable.

Although these compounds can be combined and administered together, the radioprotection resulted from this combination cannot be predicted. In some cases, the radioprotective properties of one compound are not altered when this compound is combined with another compound. For instance, as shown in Figure 2C, the administration of PT produces 40% mice survival 60 days after application of radiation. Delphinidin provides around 12% survival. However, when PT is combined with Delphinidin, the resulting survival remains around 40%, which is approximately the survival provided by the PT compound alone. In this case, the effect of Delphinidin is not added to the effect of PT.

In other cases, surprisingly, the radioprotective effect of a compound is negatively affected by the co-administration of another compound. This is shown in Figure 2C, where the radioprotective effect of PT provides a 40% survival after 60 days, and Gallic acid provides around 5% protection. However, when both compounds are combined, the overall rate of survival is around 35%, resulting in a reduced radioprotective effect than the administration of PT alone.

In other cases, the radioprotective effect of two compounds is summed when they are combined, resulting in a protection derived of their individual effects added together. For instance, in Figure 2C it is shown that the protection provided by PT is around 40%, and the protection provided by Genistein is around 7%. Thus, when both compounds are combined, the resulting protection amounts to around 45%, which means that both compounds are exerting their effect and the overall protection is, more or less, the sum of the effects of both compounds.

More interestingly, the authors of the present invention have surprisingly found that, not only the combination of two compounds can result in no changes, or detrimental, or accumulative protection, but also, in very few cases, the combination of two compounds results in enhanced radioprotective properties that go beyond the mere accumulation of their effect. That is, the specific combinations of specific compounds provide an enhanced protection that cannot be explained by the accumulation of their individual effects, which means that there is a synergistic effect among them. This is shown in Figure 2B and 2C. In figure 2C, Silibinin (SIL) provides around 10% survival and PT provides around 40% survival, but the combination of both of them provides more than 70% survival. This clearly shows that the combination of SIL and PT does not merely produce an added effect of their radioprotective properties, but also produces a synergistic effect resulting in an enhanced radioprotection.

These findings suggest that, to obtain enhanced radioprotection, care should be taken with the compounds that are combined, since some combinations are able to enhance the radioprotection, while other combinations are detrimental, and in this later case it would be more advisable to administrate the compounds individually.

Thus, in order to obtain an enhanced radioprotection, one cannot just arbitrarily combine compounds that have different radioprotective properties, since this combination does not always result in an overall enhanced radioprotection. Depending on which compounds are co-administrated, the resulting radioprotection can be worse, can be the same or the sum of the two compounds, but that also, some compounds combined together provide with a synergistic effect and an enhanced protection that is not derivable from the mere sum of their radioprotective properties.

Another aspect that was found by the inventors is that the specific combination of PT and SIL provide protection for long periods of time. This is surprising because, as stated above, not all compounds exert a radioprotective effect for more than 30 days, see, for instance, EGCG, Quercetin, Naringerin or Phloridzin compounds in Figure 2B (30 days), where their radioprotective effect is reduced to 0% after 60 days (Figure 2C). However, as shown in Figure 3, the combination of PT and SIL provided around 25% survival (5 mice alive out of 20) at day 360, that is, after a year. Thus, the combination of PT and SIL not only results in a synergistic enhanced protective effect, but this effect is also durable. Additionally, as shown in Figure 11B, when PT and SIL are combined with other radiomitigator compounds, such as nicotinamide riboside (NR) or fibroblast-stimulating lipopeptide-1 (FSL-1), the survival is even further increased to up to 50-60% after a year and, remarkably, when the four compounds (PT, SIL, NR and FSL-1) are combined, they provide a nearly full protection (90%) of the animals after 360 days.

In conclusion, the present invention provides two main findings: on the one hand, it was found that the co-administration of PT and SIL, two natural compounds with radioprotective properties, provide an enhanced and durable synergistic radioprotection not derived from the mere accumulation of their corresponding radioprotective effects. On the other hand, it was also found that the combination of these two compounds with a radiomitigator compound, such as NR and/or FSL-1, can further enhance the protection to radiation up to 90% of survival for long period of times, even for a year.

In order to study in detail the synergistic effect of SIL and PT, the authors further investigated the potential signaling pathways that may be involved in the protective effect elicited by the combination of PT+SIL. As shown in Fig. 15a, as compared to controls, both PT and SIL increased nuclear Nrf2 while decreasing nuclear NF-κB levels. These effects, as a whole, suggest a cooperative effect in promoting the antioxidant defenses while downregulating the NF-κB-dependent proinflammatory response. pPGC-1α was also increased by PT (Fig. 15a). Interestingly, although SIL alone did not affect Sirt1 levels, the combination of the two polyphenols increased Sirt1 levels more than PT alone (Fig. 15a). These results lead to the conclusion that the synergistic effect of PTE and SIL is based on the cooperative effect that both compounds have in alleviating oxidative stress and DNA damage occurring in cells, and open the path for the exploitation of compositions comprising both compounds for use in the fields of cosmetics, nutrition, nutraceutics, and medicine.

### Pterostilbene for use in the prevention, amelioration or reduction of diseases induced by ionizing radiation.

A **first aspect** of the present invention relates to a Stilbenoid, preferably Resveratrol or Pterostilbene, or a pharmaceutically acceptable salt thereof, for use in the treatment, prevention, amelioration or reduction of diseases induced by ionizing radiation in a subject, in combination with Silibinin, or a pharmaceutically acceptable salt thereof. Preferably, the Stilbenoid used is Pterostilbene or a pharmaceutically acceptable salt thereof. Thus, in a first aspect, the present invention provides Pterostilbene, or a pharmaceutically acceptable salt thereof, for use in the treatment, prevention, amelioration or reduction of diseases induced by ionizing radiation in a subject, in combination with Silibinin, or a pharmaceutically acceptable salt thereof, wherein the administration is carried out before the radiation, or after the radiation, or during the radiation; and wherein the combination is herein understood as the simultaneous or sequential administration, in any order, of a) Pterostilbene, or a pharmaceutically acceptable salt thereof, and b) Silibinin, or a pharmaceutically acceptable salt thereof, to said subject. In a particular embodiment, the diseases induced by ionizing radiation are related to DNA damage, radiation-induced cytotoxicity, genotoxicity and/or oxidative cellular damage. In a preferred embodiment, the diseases induced by the ionizing radiation are acute radiation syndrome and/or chronic radiation syndrome. In another embodiment, said diseases are selected from the group consisting of skin diseases, acute radiation syndrome, cancer and cardiovascular diseases.

Under the first aspect of the present invention, Pterostilbene is used in combination with Silibinin. PT and SIL may be administered in a sole composition or in separate compositions.

In a preferred embodiment, the Pterostilbene is Pterostilbene phosphate disodium salt. In another preferred embodiment, the Silibinin is silibinin-C-2',3-dihydrogen succinate, disodium salt.

As stated above, PT and SIL, or any pharmaceutically acceptable salt or derivatives thereof, are administered in combination to prevent, ameliorate or reduce diseases induced by ionizing radiation in a subject. In a preferred embodiment, the subject is a mammal. More preferably, the subject is a human being.

### Administration schedule and route

The administration can be simultaneous (at the same time) or sequential. "Sequential administration" as used herein refers to the administration of SIL or a composition comprising thereof shortly before or after administration of PT or a composition comprising thereof. "Shortly before or after" as used herein is understood as within an interval of 1 to 24 hours, preferably 1 to 12 hours, preferably 1 to 3 hours, preferably within 2 hours, more preferably within 1 hour or less, such as within 45 minutes, 30 minutes, 15 minutes or less. In an embodiment, PT is administered first followed by SIL. In another embodiment, SIL is administered first followed by PT. In a preferred embodiment, PT and SIL administration is carried out simultaneously. In an embodiment, the PT and SIL are administered in combination simultaneously, as a part of a single composition or as part of two different compositions.

In an embodiment, said pharmaceutically acceptable compounds and compositions are formulated to be compatible with its intended route of administration. Methods to accomplish the administration are known to those of ordinary skill in the art. This includes, for example, injections, by parenteral routes such as intravascular, intravenous, intraarterial, subcutaneous, intramuscular, intraperitoneal, intraventricular, intraepidural, or others as well as oral, topical, nasal, ophthalmic, rectal, or topical. Sustained release administration is also specifically contemplated, by such means as depot injections or erodible implants. A preferred route of administration is parenteral administration, which is herein understood as the administration within a vessel or vessels and typically includes intravenous or intraarterial administration. Currently, the most preferred route of administration is parenteral, oral or topical.

The administration can be carried out only once, or there can be more than one administration. In a preferred embodiment, there compounds are administered several times. In a particular embodiment, the administration may comprise at least one, two, three, four, five, six, seven or more doses.

The administration can be performed before, during or after the radiation occurs. For example, the administration can be performed at least 10, 9, 8, 7, 6, 5, 4, 3, 2, 1 days before the radiation occurs. In another embodiment, the administration occurs after radiation. In an embodiment, the administration carried out after the radiation occurs 1, 2, 3, 4, 5, 6, 7, 8, 9, 10 or more than 10 days, such as 20 or 30 days, or more than one moth, such as 2, 3, 5, 6, 8, 9 months after radiation. In a particular embodiment, the administration is carried out on the same day where the radiation occurs, either before or after it. In an embodiment, the administration consists of several doses administered during several days before, on the same day, and/or after the radiation occurs. In a preferred embodiment, Pterostilbene and Silibinin, or any pharmaceutically acceptable salts thereof, are administrated several times simultaneously or sequentially before and/or after radiation, or both.

### Dosage

The optimal dose will be selected according to the administration route, treatment regime and/or administration schedule, having regard to the existing toxicity and effectiveness data. In a preferred embodiment, PT and SIL for use according to the first aspect of the present invention are administered in a dosage capable of providing a radioprotective effect. As defined above, "radioprotection" is understood the ability to reduce the direct damage caused by radiation. Accordingly, in the present invention, the dosage of PT and SIL is not particularly restricted.

Further, it will be evident for a skilled person to convert the doses applied to mouse models as disclosed herein to the equivalent dose to be applied in humans. To do so, the skilled person may consult the well-known common practice guide for dose conversion between animals and humans, see, e.g., Nair AB, Jacob S. A simple practice guide for dose conversion between animals and human. J Basic Clin Pharma 2016;7:27-31. Briefly, therapeutic doses in mouse models can be converted to humans by dividing them by about 12.3. That is, if a dose of 100 mg/kg presented a therapeutical effect in mice, the human equivalent dose would be 8.13 mg/kg.

In in an embodiment, PT, or a pharmaceutically acceptable salt thereof, is administered at a dosage up to 500 mg/kg. PT may be administered at dosages ranging from 300 mg/kg to 30 mg/kg. Preferably, PT is administered at a dosage from 200 mg/kg to 40 mg/kg or 150 mg/kg to 40 mg/kg. More preferably, PT is administered at a dosage of 100 mg/kg. In in an embodiment, PT, or a pharmaceutically acceptable salt thereof, is administered to a human at a dosage up to 40.65 mg/kg. PT may be administered to humans at dosages ranging from 24,39 mg/kg to 2.43 mg/kg. Preferably, PT is administered to humans at a dosage from 16.2 mg/kg to 3.25 mg/kg or 12.19 mg/kg to 3.25 mg/kg. More preferably, PT is administered to humans at a dosage of 8.13 mg/kg.

In another embodiment, SL, or a pharmaceutically acceptable salt thereof, is administered at a dosage up to 500 mg/kg. SIL may be administered at a dosage ranging from 200 mg/kg to 35 mg/kg. Preferably, SIL is administered at a dosage from 150 mg/kg to 40 mg/kg or 100 mg/kg to 50 mg/kg. More preferably, PT is administered at a dosage of 70 mg/kg or the equivalent for humans. In another embodiment, SL, or a pharmaceutically acceptable salt thereof, is administered to humans at a dosage up to 40.65 mg/kg. SIL may be administered to humans at a dosage ranging from 16.26 mg/kg to 2.84 mg/kg. Preferably, SIL is administered to humans at a dosage from 12.19 mg/kg to 3.25 mg/kg or 8.13 mg/kg to 4.06 mg/kg. More preferably, PT is administered to humans at a dosage of 5.69 mg/kg or the equivalent for humans.

### Administration of further compounds

In an embodiment of the first aspect, the use described in any of the above mentioned embodiments further comprises the administration in combination with at least one further radiomitigator compound, wherein the use described herein is herein understood as the simultaneous or sequential administration of a) Pterostilbene, or a pharmaceutically acceptable salt thereof, b) Silibinin, or a pharmaceutically acceptable salt thereof, and c) one or more radiomitigator compounds, to said subject. In a preferred embodiment, the at least one or more radiomitigator compound/s refers to a nicotinamide adenine dinucleotide booster (referred from now on as NAD+ boosters), and/or a Fibroblast-Stimulating Lipopeptide-1 (referred from now on as FSL-1), or any pharmaceutically acceptable salt thereof. Please note that, in the context of the present invention, although FSL-1 is preferred, FSL-1 is a type of Fibroblast-Stimulating Lipopeptide and can be thus replaced by any other Fibroblast-Stimulating Lipopeptide. Consequently, the term "FSL-1" as used in any of the embodiments of the present invention can be thus replaced by any other suitable Fibroblast-Stimulating.

As defined above, a "radiomitigator" is a compound that is able to minimize toxicity after even after radiation has been delivered. Thus, in an embodiment of the first aspect, the administration of PT combined with SIL further comprises the administration of one or more radiomitigator compound/s. The combinations between PT, SIL and the one or more radiomitigator compound/s are not limited, that is, PT can be administered in a single composition together with SIL and one or more radiomitigator/s, or they can be administered individually in different compositions. All the possible combinations of the three (or four or more) compounds are included herein.

The administration routes and schedules described above also apply in the case of PT and SIL administered together with one or more radiomitigator compound/s.

In a preferred embodiment, the use of PT in the prevention, amelioration or reduction of diseases induced by ionizing radiation in a subject, in combination with SIL, or their salt thereof, further comprises the administration of a NAD+ booster. Thus, in this embodiment, the at least three compounds are administered together, and can be comprised in the same or in different compositions and can be administered sequentially or simultaneously.

In another preferred embodiment, the use of PT in the prevention, amelioration or reduction of diseases induced by ionizing radiation in a subject, in combination with SIL, their salt thereof, further comprises the administration of FSL-1. Thus, in this embodiment, the at least three compounds are administered together, and can be comprised in the same or in different compositions and can be administered sequentially or simultaneously.

In an even more preferred embodiment, the use of PT in the prevention, amelioration or reduction of diseases induced by ionizing radiation in a subject, in combination with SIL, their salts thereof, further comprises the administration of both a NAD+ booster and FSL-1. Thus, in this embodiment, the four compounds are and can be comprised in the same or in different compositions and can be administered sequentially or simultaneously.

In a more preferred embodiment, the NAD+ booster is Nicotinamide riboside or 1-(β-D-Ribofuranosyl)nicotinamide (referred from now on as NR), a pharmaceutically acceptable salt or a derivative thereof.

In an even more preferred embodiment, the use of PT in the prevention, amelioration or reduction of diseases induced by ionizing radiation in a subject, in combination with SIL, their salts thereof, further comprises the administration of NR and FSL-1. Thus, in this embodiment, the four compounds are administered together, in one or more than one doses, comprised in the same or in different compositions, and administered sequentially or simultaneously before, during or after the radiation.

In a preferred embodiment, PT and SIL are administered once per day during several days before the radiation and/or several days after the radiation. More preferably, PT and SIL are administered daily during 10, 9, 8, 7, 6, 5, 4, 3, 2, 1, preferably 5, days before the radiation. In another embodiment, PT and SIL are administered daily before the radiation and up to 5, 4, 3, 2, 1 days after the radiation. Preferably, PT and SIL are administered daily during the 5 previous days before the radiation and during the 2 days after radiation.

In another embodiment, the NAD+ booster is administered once per day on the same day of the radiation and during the next month after radiation. In another embodiment, the NAD+ booster is administered at least 60 mins after the radiation and it is further administered during the next 30 days after the radiation.

In another embodiment, the FSL-1 is administered only once, preferably after the radiation, more preferably at least one day after the radiation.

In an embodiment, the NAD+ booster, or a pharmaceutically acceptable salt thereof, is administered at a dosage up to 500 mg/kg. In an embodiment, the NAD+ booster, or a pharmaceutically acceptable salt thereof, is administered at a dosage from 400 mg/kg to 100mg/kg. Preferably, the NAD+ booster is administered at a dosage from 300 mg/kg to 150 mg/kg or 200 mg/kg to 150 mg/kg. More preferably, the NAD+ booster is administered at a dosage of 185 mg/kg. In an embodiment, the NAD+ booster, or a pharmaceutically acceptable salt thereof, is administered to humans at a dosage up to 40.65 mg/kg. In an embodiment, the NAD+ booster, or a pharmaceutically acceptable salt thereof, is administered to humans at a dosage from 32.52 mg/kg to 8.13 mg/kg. Preferably, the NAD+ booster is administered to humans at a dosage from 24.39 mg/kg to 12.19 mg/kg or 16.26 mg/kg to 12.19 mg/kg. More preferably, the NAD+ booster is administered to humans at a dosage of 15.04 mg/kg.

In another embodiment, the NAD+ booster is Nicotinamide Riboside, or any pharmaceutically acceptable salt thereof, and it is administered at a dosage from 400 mg/kg to 100mg/kg. Preferably, the Nicotinamide Riboside is administered at a dosage from 300 mg/kg to 150 mg/kg or 200 mg/kg to 150 mg/kg. More preferably, the Nicotinamide Riboside is administered at a dosage of 185 mg/kg. In another embodiment, the NAD+ booster is Nicotinamide Riboside, or any pharmaceutically acceptable salt thereof, and it is administered to humans at a dosage from 32.52 mg/kg to 8.13 mg/kg. Preferably, the Nicotinamide Riboside is administered to humans at a dosage from 24.39 mg/kg to 12.19 mg/kg or 16.26 mg/kg to 12.19 mg/kg. More preferably, the Nicotinamide Riboside is administered to humans at a dosage of 15.04 mg/kg.

In in an embodiment, FSL-1, or a pharmaceutically acceptable salt thereof, is administered at a dosage up to 1mg/kg. In another embodiment, the FSL-1 compound, or a pharmaceutically acceptable salt thereof, is administered at a dosage from 1 mg/kg to 0.1 mg/kg. Preferably, the FSL-1 compound is administered at a dosage from 0.75 mg/kg to 0.1 mg/kg or 0.5 mg/kg to 0.1 mg/kg. More preferably, FSL-1 compound is administered at a dosage of 0.25 mg/kg. In in an embodiment, FSL-1, or a pharmaceutically acceptable salt thereof, is administered at a dosage up to 0.08 mg/kg. In another embodiment, the FSL-1 compound, or a pharmaceutically acceptable salt thereof, is administered at a dosage from 0.08 mg/kg to 0.008 mg/kg. Preferably, the FSL-1 compound is administered at a dosage from 0.06 mg/kg to 0.008 mg/kg or 0.04 mg/kg to 0.081 mg/kg. More preferably, FSL-1 compound is administered at a dosage of 0.020 mg/kg.

In an embodiment, the ionizing radiation is gamma (γ) rays or X-rays.

### A composition comprising PT and SIL

In a **second aspect,** the present invention relates to a composition comprising a) a Stilbenoid, preferably Resveratrol or Pterostilbene or a salt thereof, and b) Silibinin, or a salt thereof. Preferably, the Stilbenoid is Pterostilbene or a salt thereof. Thus, a second aspect of the invention provides a composition comprising a) Pterostilbene, or a salt thereof, and b) Silibinin, or a salt thereof. Preferably, the composition is a pharmaceutical, nutraceutical, cosmetic or food composition. For the purposes of the present invention the term nutraceutical means a "food-drug", i.e. a health food that associates nutritional components with the curative properties of natural active ingredients of proven and recognised effectiveness, in this case PT and SIL.

Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, that the preparation will contain a pharmaceutically acceptable excipient that serves as a stabilizer, particularly for peptide, protein or other like molecules if they are to be included in the vaccine composition. Examples of suitable carriers include, without limitation, pharmaceutical grades of dextrose, sucrose, lactose, trehalose, mannitol, sorbitol, inositol, dextran, and the like. Other suitable carriers include, starch, cellulose, sodium or calcium phosphates, citric acid, tartaric acid, glycine, high molecular weight polyethylene glycols (PEGs), and combination thereof. A thorough discussion of pharmaceutically acceptable excipients, additives and carriers is available in Remington's Pharmaceutical Sciences (22nd edition, 2012).

Different options regarding pharmaceutical formulations, structural modifications and delivery systems, can help to facilitate the oral administration of PTE and SIL and are also included herein. Some examples include prodrugs (carboxyesters, sulfonates, sulfates, phosphates, acetals, or carbamates or carbonates), nanoemulsions, polyionic/polymeric shells encapsulating nanoparticles, liposomes, or exosomes. In particular, any pharmaceutically acceptable formulation of PTE and SIL is included herein.

The composition or compositions according to the second aspect comprise PT and SIL in the dosages defined in the first aspect of the invention and it is administered according to the administration route and schedules defined under the first aspect of the invention. Further details and preferred embodiments on the dosage, route and schedules of administration of said composition are also provided under the first aspect of the invention.

Said composition comprise PT and SIL, or any salts thereof, and may further comprise one or more radiomitigator compound/s, as defined under the first aspect of the invention. As stated above, in an embodiment, the radiomitigator compound may be NAD+ booster and/or FSL-1. In a preferred embodiment, the composition comprising PT and SIL further comprises at least two radiomitigator compounds, preferably NR and FSL-1.

Preferably, said composition or compositions is in a form suitable for parenteral, oral or topic administration. In a preferred embodiment, said composition is an aqueous composition, more preferably a stable aqueous composition. As used herein, a "stable composition" may refer to a formulation in which the active ingredient therein essentially retains its physical stability and/or chemical stability and/or biological activity upon storage.

The composition or compositions of the second aspect can be a food composition. The food composition can be a liquid or a solid composition. In the context of the present invention, such liquid composition includes, but is not limited to, any beverage selected from the group consisting of animal or plant milk, as well as any derivative thereof, such as for example, milk shakes, yogurt, kefir, etc.; fruit and/or vegetable juices; still water or sparkling water, or flavored or sweetened water or beverages (by means of nutritive sweeteners (sucrose, fructose...) or artificial sweeteners); seasonings, alcoholic beverages of any type; tea, coffee; as well as all types of refreshing beverages or soft drinks, or energizing beverages.

The food composition can also be a solid composition. Such solid composition can, for example, be selected from, but is not limited to, the group consisting of animal or plant milk derivatives, such as cheese, butter, margarine and tofu; any type of bread, including fresh, packaged or frozen bread, sliced bread, wholemeal bread, spiced bread, sweet bread, salty bread, etc; pasta prepared from any cereal flour, such aswheat or semolina flour (macaroni, spaghetti, noodles, etc); baked goods; infusions, tea or coffee, in bulk or insachets, for preparing beverages; jellies, candies, including gummy candies, better known as "soft fruit candies"; as well as any type of solid seasoning, or mixtures thereof.

Finally, the food composition can also be a nutritional, dietary or food supplement or complement. These terms are normally used for compositions consumed orally, which contain an ingredient intended for complementing the diet, in the case of the present invention, the PT and SIL or any of their salts or derivatives. They shall never replace a conventional food or be the only component of a meal or of the diet. The composition may also be nutraceutical compositions. The composition may be further used in the preparation of fuctional food or food that have an additional function upon addition of PTE in combination of SIL, as defined throughout the description. Nutritional, dietary or nutraceutical compositions or food supplements or functional food can be found in different presentations, such as pastilles, pills, tablets, capsules, soft gelatin capsules, gelatin capsules, wafers, effervescent tablets, liquids (solution, suspension, syrup), granules and powders, all of which are included as particular embodiments within the scope of the present invention. Dietetically or pharmaceutically acceptable excipients are obvious for the skilled person for obtaining any of the preceding presentations, and they are included within the scope of the present invention.

The present invention further relates to said composition for use as defined in the first aspect of the invention.

### A kit of parts comprising PT and SIL

In a **third aspect,** the present invention relates to a kit of parts comprising at least two recipients comprising at least a) a Stilbenoid, preferably Resveratrol or Pterostilbene, and Silibinin, or any salts thereof, and optionally at least one or more radiomitigator compounds as defined above. Preferably, the Stilbenoid comprised in the kit of parts is Pterostilbene.

Typically, the two or more ingredients are provided formulated as a composition and provided in two or more separate recipients. For instance, PT and SIL can be combined in one recipient, and the radiomitigators in the other. In an embodiment, each compound is contained in a separate recipient. In further embodiments, the radiomitigator compounds are present in similar recipients as the radiomitigator compounds.

Preferably, said compositions are as defined in the previous aspects of the invention, for use as defined in the previous aspects of the invention, preferably in the therapeutic treatment of a disease induced by ionizing radiation.

### Method for preventing, ameliorating or reducing diseases induced by ionizing radiation.

In **a fourth aspect,** the present invention relates to a method for preventing, treating, including ameliorating or reducing, diseases induced by ionizing radiation in a subject, comprising administering to a subject a Stilbenoid, preferably Resveratrol or Pterostilbene or a pharmaceutically acceptable salt thereof, in combination with Silibinin, or any pharmaceutically acceptable salt thereof. Preferably, the Stilbenoid is Pterostilbene. Thus, in a fourth aspect, the present invention provides the composition for preventing, treating, including ameliorating or reducing, diseases induced by ionizing radiation in a subject, comprising administering Pterostilbene in combination with Silibinin, or any pharmaceutically acceptable salts thereof, wherein said administration is performed before the radiation, or after the radiation, or during the radiation; and wherein the combination is herein understood as the simultaneous or sequential administration of a) Pterostilbene, or a pharmaceutically acceptable salt thereof, b) Silibinin, or any pharmaceutically acceptable salt thereof, and optionally, c) at least one or more radiomitigator compounds, as defined throughout the present specification, to said subject. Similar as described above in the first and second aspects of the present invention, the at least one or more radiomitigator compounds may be NAD+ boosters and/or FSL-1.

This fourth aspect is related to the first and second aspects described above. Thus, the compounds and the pharmaceutical compositions, administration dosages, routes and schedules are provided under the previous aspects of the invention and apply herein.

### Pterostilbene in combination with Silibinin for use in nutraceutical field or as a nutraceutical composition.

In a **fifth aspect,** the present invention further relates to the use of a Stilbenoid, preferably Resveratrol or Pterostilbene, in combination with Silibinin, or any salt thereof, or the use of the composition according to the second aspect, wherein said use in the nutraceutical field or as a nutraceutical composition. Preferably, the Stilbenoid used is Pterostilbene. Thus, the fifth aspect provides Pterostilbene, or a salt thereof, in combination with Silibinin, or a salt thereof, for use in the nutraceutical field or as a nutraceutical composition, wherein the combination is herein understood as the simultaneous or sequential administration, in any order, of a) Pterostilbene, or a salt thereof, and b) Silibinin, or a salt thereof, to said subject.

In a preferred embodiment, the use as a nutraceutical composition is for the treatment or prevention of diseases induced by ionizing radiation, for the treatment or prevention of neurodegenerative, cardiovascular, pulmonary, metabolic, or aged-related disorders, and/or for the protection or repairment of the adverse effects of the sun on human skin or lips. The term "repairing the adverse effects of the sun on human skin or lips" is used herein to designate arresting, reversing, ameliorating, diminishing, and/or reducing defects, imperfections, or aesthetically unpleasant conditions of the skin, which include, but are not limited to: age spots, sunburn, sun spots, lines, fine lines, wrinkles, crow's feet, spider veins, stretch marks, dark eye circles, hyperpigmentation, hypopigmentation, discoloration, uneven skin tone, dullness, freckles, skin breakout, blemishes, skin fragility, dryness, patchiness, tactile roughness, chapping, sagginess, thinning, enlarged pores, cellulite formation, acne formation, rosacea, psoriasis, and eczema. The term "skin' includes facial or body skin.

Preferably, the diseases induced by ionizing radiation are related to DNA damage, radiation-induced cytotoxicity, genotoxicity and/or oxidative cellular damage. More preferably, the diseases induced by ionizing radiation are selected from the list consisting of acute radiation syndrome and/or chronic radiation syndrome.

Preferably, the nutraceutical composition is used in the treatment of a disorder selected from the list consisting of Alzheimer or other dementias or cognitive impairment associated to aging, Parkinson, Huntington disease, Multiple sclerosis, Kennedy disease (Spinal and bulbar muscular atrophy), kidney disease, cerebrovascular disease, hypertension, Chronic obstructive pulmonary disease, Amyotrophic lateral sclerosis and Diabetes.

This fifth aspect is related to the first and second aspects described above. Thus, the compounds and the pharmaceutical compositions, administration dosages, routes and schedules are provided under the previous aspects of the invention and apply herein.

### Pterostilbene in combination with Silibinin for the non-therapeutical use as a cosmetic composition

In a **sixth aspect,** the present invention further relates to the non-therapeutic cosmetic use of a Stilbenoid, preferably Resveratrol or Pterostilbene, in combination with Silibinin, or any salt thereof. Further, the sixth aspect also relates to the non-therapeutic cosmetic use of the composition according to the second aspect. Preferably, the Stilbenoid used is Pterostilbene. Thus, the sixth aspect of the present invention provides Pterostilbene, or a salt thereof, in combination with Silibinin, or a salt thereof, for the non-therapeutic cosmetic use or as a cosmetic composition, wherein the combination is herein understood as the simultaneous or sequential administration, in any order, of a) Pterostilbene, or a salt thereof, and b) Silibinin, or a salt thereof, to said subject.

As used herein, the expression "non-therapeutic cosmetic use" refers to a method used to improve a person's appearance, i.e., to beautify appearance. The used in cosmetics does not involve a treatment by surgery or therapy. The cosmetic care includes the topical application of PTE and SIL on the cutaneous areas to be treated. Preferably, the cosmetic use of the composition is with the non-therapeutic purposes of cleaning, beautifying, adding to the attractiveness, altering the appearance, or keeping or promoting the skin or hair in good condition. Said purposes may also be whitening, minimizing the appearance of lines in the face and body, protecting from the sun and sun rays. In an embodiment, the non-therapeutic cosmetic use is to prevent, reduce and/or ameliorate the natural process of skin aging.

Preferably, the non-therapeutic use is to protect, ameliorate and/or reduce the adverse effects of the sun on human skin and lips, including, but not limiting to, age spots, sunburn, sun spots, lines, fine lines, wrinkles, crow's feet, spider veins, stretch marks, dark eye circles, hyperpigmentation, hypopigmentation, discoloration, uneven skin tone, dullness, freckles, skin breakout, blemishes, skin fragility, dryness, patchiness, tactile roughness, chapping, sagginess, thinning, enlarged pores. The term "skin' includes facial or body skin.

This sixth aspect is related to the first and second aspects described above. Thus, the compounds and the compositions, administration dosages, routes and schedules compatible with a cosmetic use and provided under the previous aspects of the invention also apply herein.

### Pterostilbene in combination with Silibinin for use to increase the intracellular sirtuin levels.

Based on the data reported in Figs. 5-8, PT+SIL was shown to exert protection against the induced oxidative damage. Further, as shown in Fig. 15, it was found that this mechanism is directly associated with a synergistic increase caused by PTE+SIL in Sirtuins (Sirt) levels, especially Sirt1. Sirtuins are a family of signaling proteins involved in metabolic regulation as they control many vital functions, being also involved in several pathologies such as metabolic, cardiovascular, pulmonary diseases, neurodegenerative and age-related disorders, and cancer. Sirtuins have been shown to be modulators of inflammation and autophagy. A growing body of evidence suggests that sirtuin-activating or increasing compounds show promise as therapeutic approaches for treating metabolic dysfunction, cancer, and age-related diseases, strongly supporting the use of PTE in combination with SIL for use in the treatment or prevention of said diseases. In particular, several studies have showed the role of Sirtuins in neuroprotection, especially in neurodegenerative disorders such as Alzheimer or other dementias or cognitive impairment associated to aging, Parkinson, Huntington disease, Multiple sclerosis, Kennedy disease (Spinal and bulbar muscular atrophy), Amyotrophic lateral sclerosis, and other neuronal-related diseases (see Kim D, et al. SIRT1 deacetylase protects against neurodegeneration in models for Alzheimer's disease and amyotrophic lateral sclerosis. EMBO J. 2007;26(13):3169-3179; Donmez G et al. SIRT1 protects against α-synuclein aggregation by activating molecular chaperones. J Neurosci. 2012;32(1):124-132; Outeiro TF et al. Sirtuin 2 inhibitors rescue alpha-synuclein-mediated toxicity in models of Parkinson's disease. Science. 2007;317(5837):516-519; Pallos J et al. Inhibition of specific HDACs and sirtuins suppresses pathogenesis in a Drosophila model of Huntington's disease. Hum Mol Genet. 2008;17(23):3767-377; Luthi-Carter R et al. SIRT2 inhibition achieves neuroprotection by decreasing sterol biosynthesis. Proc Natl Acad Sci U S A. 2010;107(17):7927-7932; Shindler KS, et al. SIRT1 activation confers neuroprotection in experimental optic neuritis. Invest Ophthalmol Vis Sci. 2007;48(8):3602-3609; Montie HL, et al. SIRT1 modulates aggregation and toxicity through deacetylation of the androgen receptor in cell models of SBMA. J Neurosci. 2011;31(48):17425-17436). In view of this, in **a seventh aspect,** the present invention a Stilbenoid, preferably Resveratrol or Pterostilbene, in combination with Silibinin, or any salt thereof, or a composition according to the second aspect, for use to increase the intracellular levels of Sirtuins in a patient in need thereof, wherein said increase is as compared to the intracellular levels of Sirtuins in the same patient before treatment with the Stilbenoid, preferably Resveratrol or Pterostilbene in combination with Silibinin, or any salt thereof, or before treatment with the composition according to the second aspect. Preferably, the Stilbenoid is Pterostilbene. Preferably, the Sirtuin which intracellular levels are increased is Sirt1, Sirt, 2 or Sirt3, most preferably Sirt1. The increase in the intracellular Sirtuin levels as compared to an untreated patient is preferably a statistical significant increase, and it can be measured by any suitable technique aimed at measuring the amount of a protein, such as, but not limited to, Bradford assay, Colorimetric protein measurement BCA, antibody-based assays, such as Western blot or ELISA, Lowry protein assay, fluorescence-based assays, protein mass-spectrometry based assays, nanoparticles and nanopore-based methods, etc.

By "statistically significant increase of the intracellular levels of Sirtuins" is referred herein as the determination by an analyst that the increase in the expression or activation levels of said proteins (in this case, Sirtuins) is not explainable by chance alone. Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant. For example, the increase of the intracellular levels of Sirtuins is statistically significant when a statistical test is performed to compare it to the basal intracellular levels of Sirtuins in a reference or untreated cell, and wherein the resulting p-value of said statistical test is of 0.1 or lower, preferably 0.05, 0.01, 0.001 or lower.

Preferably, the patient in need thereof is suffering from a disease or disorder characterized by a reduction in the intracellular levels of Sirtuins, preferably a reduction in Sirt1 intracellular levels. Preferably, the patient in need thereof is suffering from a neurodegenerative, cardiovascular, pulmonary, metabolic, or aged-related disorders characterized by a reduction in the intracellular levels of Sirtuins, preferably Sirt1, as compared to the intracellular levels of Sirtuins in a healthy person. Preferably, the disorder is selected from the list consisting of Alzheimer or other dementias or cognitive impairment associated to aging, Parkinson, Huntington disease, Multiple sclerosis, Kennedy disease (Spinal and bulbar muscular atrophy), kidney disease, cerebrovascular disease, hypertension, Chronic obstructive pulmonary disease, Amyotrophic lateral sclerosis and Diabetes.

Preferably, Pterostilbene, or a salt thereof, in combination with Silibinin, or a salt thereof, is used in the treatment, prevention, amelioration or reduction of neurodegenerative diseases, wherein the combination is herein understood as the simultaneous or sequential administration, in any order, of a) Pterostilbene, or a salt thereof, and b) Silibinin, or a salt thereof, to said subject. Preferably, said disorder is characterized by a reduction in the intracellular levels of Sirtuins, preferably Sirt1, as compared to a healthy person. In an embodiment, the neurodegenerative disorder is selected for the group consisting of Alzheimer or other dementias or cognitive impairment associated to aging, Parkinson, Huntington disease, Multiple sclerosis, Kennedy disease (Spinal and bulbar muscular atrophy), kidney disease, cerebrovascular disease, hypertension, Chronic obstructive pulmonary disease, Amyotrophic lateral sclerosis and Diabetes.In an embodiment, the cell in which the intracellular levels of Sirtuin, preferably Sirt1, are increased upon treatment is from nervous tissue. Preferably, the cell is a neuron or neuroglia.

Additionally, it is also known that Sirtuins are potential therapeutic targets in the treatment of metabolic disorders, such as diabetes. Several studies suggest that overexpression of Sirt1 in pancreatic cells can be used to control of glucemia in diabetic patients (see Nerurkar et al. Respected Sir(2): magic target for diabetes. Cellscience. 2008;4(4):82-96; Moynihan et al. Increased dosage of mammalian Sir2 in pancreatic beta cells enhances glucose-stimulated insulin secretion in mice. Cell Metab. 2005;2(2):105-117, and Bordone et al. Sirt1 regulates insulin secretion by repressing UCP2 in pancreatic beta cells. PLoS Biol. 2006;4(2):e31). In view of this and based on the findings provided herein, a Stilbenoid, preferably Resveratrol or Pterostilbene, in combination with Silibinin, or the composition according to the second aspect, can be also used in the treatment, prevention, amelioration or reduction of diabetes. Preferably, the Stilbenoid is Pterostilbene. Thus, in a preferred embodiment, the present invention also provides Pterostilbene, or a salt thereof, in combination with Silibinin, or a salt thereof, for use in the treatment, prevention, amelioration or reduction of diabetes, wherein the combination is herein understood as the simultaneous or sequential administration, in any order, of a) Pterostilbene, or a salt thereof, and b) Silibinin, or a salt thereof, to said subject.

This seventh aspect is related to the first and second aspects described above. Thus, the compounds and the pharmaceutical compositions, administration dosages, routes and schedules are provided under the previous aspects of the invention and apply herein.

Similar as defined in the first aspect, and also included as embodiments in the fifth, sixth, and seventh aspects, the PT in combination with SIL may be administered in a sole composition or in separate compositions. In embodiments of the fifth, sixth, and seventh aspects, the Pterostilbene is Pterostilbene phosphate disodium salt. In other embodiments of said aspects, the Silibinin is silibinin-C-2',3-dihydrogen succinate, disodium salt. It is noted that the uses described in any of the above mentioned aspects further comprises the administration of PTE and SIL in combination with at least one further radiomitigator compound, wherein the use described herein is understood as the simultaneous or sequential administration of a) Pterostilbene, or a salt thereof, b) Silibinin, or a salt thereof, and c) one or more radiomitigator compounds, to said subject. In a preferred embodiment, the at least one or more radiomitigator compound/s refers to a nicotinamide adenine dinucleotide booster (referred from now on as NAD+ boosters), and/or a Fibroblast-Stimulating Lipopeptide-1 (referred from now on as FSL-1), or any salt thereof.

Similar as defined in the first aspect, and also included as embodiments in the fifth, sixth, and seventh and aspects, PT and SIL, or any salt thereof, are administered in a subject. In a preferred embodiment, the subject is a mammal. Preferably, the mammal is a human being, or a domestic animal such as dogs, cats, or horses. More preferably, the mammal is a human being.

The present invention further provides the composition for increasing the intracellular levels of Sirtuins in a subject for preventing, treating, including ameliorating or reducing diseases characterized by having low levels of Sirtuins, or decreased Sirtuin activation, as described in the previous aspects. In an embodiment, the method comprises administering Pterostilbene in combination with Silibinin, or any salt thereof, wherein the combination is herein understood as the simultaneous or sequential administration of a) Pterostilbene, or a salt thereof, b) Silibinin, or salt thereof, and optionally, c) at least one or more radiomitigator compounds, as defined throughout the present specification, to said subject. Similar as described above, the at least one or more radiomitigator compounds may be NAD+ boosters and/or FSL-1. Importantly, the dosages and administration routes defined in the first aspect also apply to the uses according to the fifth, sixth, and seventh aspects.

### EXAMPLES

### EXAMPLE 1: General Materials and Methods

Disclosed herein are general materials and methods used in the present invention. Particular procedures are further described in each of the Examples 2-4.

### Mice and treatment procedures

Swiss albino mice (male, 9-10 weeks old, 30-32 g) were obtained from Charles River Laboratories (Wilmington, MA). This strain is among the most common used in radiation studies (Williams 2010). Mice were fed with a nutritionally complete diet (standard diet, SD) before irradiation. This was A03 from Panlab (Barcelona, Spain), which has 3,200 kcal/kg and the following nutritional composition: crude protein, 23.5%; crude fat, 4.3%; crude fiber, 3.7%; and carbohydrates, 51.1%; vitamins and minerals were provided as in the NIH-7 open formula for rat and mouse. This diet meets the dietary allowances recommended for adult mice by the American Institute of Nutrition. The animals were fed *ad libitum* and kept in individual metabolic cages, in which body weight and food ingestion were monitored daily. The animal room was maintained at 22°C on a 12 h light/12 h dark cycle. *Ad libitum* intake of drinking water was also allowed. Care of irradiated mice followed the recommendation of the Robert Wood Johnson Medical School (Piscataway, NJ) for mouse total body irradiation (policy 15), which includes: a) use of antibiotics in the drinking water (134 mg ampicillin/kg/day, 40 mg baytril/kg/day, and 220/42 mg sulfamethoxazole/trimethoprim/kg/day), b) making drinking water readibly available, c) Napa nectar Systems Engineering Lab Group Inc., Napa, CA) provided on the bottom of the cage during the first 14 days and replaced daily , d) provision of softened food served in a small Petri dish on the cage floor, e) completely sterile environment (cage, food, and water) to avoid potential immunosuppression-associated infections. A freestanding wheel inside the cage was also included to facilitate the physical exercise of the animals. All experiments were started at 10.00 h to minimize possible circadian variations. Procedures involving animals were in compliance with international laws and policies (EEC Directive 86/609, OJ L 358. 1, December 12, 1987; and NIH Guide for the Care and Use of Laboratory Animals, NIH Publ. No. 85-23, 1985). Mice were subjected to g-radiation (¹³⁷Cs) (total body irradiation, TBI) in a BIOBEAM 8000 (Gamma-Service Medical GmbH, Leipzig, Germany). Single fraction radiotherapy, at total doses ranging from approx. 5.0 to 10.0 Gy, was administered at a rate of approx. 5.0 Gy/min. The irradiation distance from the source was limited to 12 cm. Radiation dosimetry was controlled using alanine dosimeters from Bruker (Billerica, MA) with dosimetry variation of +/- 0.09 Gy. For oral administration PTER and SIL (Merck Chemicals GmbH, Darmstadt, Germany) were solubilized in 2-hydroxypropyl-b-cyclodextrin, then suspended in 0.3% carboxymethylcellulose. For IP administration, a PTER salt (pterostilbene phosphate disodium salt, SYNCOM, Groningen, The Netherlands) and SIL salt (silibinin-C-2',3-dihydrogen succinate, disodium salt, Rottapharm/Madaus, Cologne, Germany) were dissolved in sterile vaccination-grade water. All other polyphenolic phytochemicals assayed (Merck Chemicals GmbH; and Cayman Chemical Co., Ann Arbor, MI) represent main flavonoids and non-flavonoids (Estrela 2017), i.e. gallic acid (a phenolic acid), caffeic acid (a hydroxycinnamic acid), curcumin (a curcuminoid), epigallocatechin gallate (a flavanol), genistein (a isoflavone), quercetin (a flavonol), luteolin (a flavone), naringenin (a flavanone), delphindin (an anthocyanidin), and phloridzin (a chalcone) were solubilized as PTER and SIL before oral administration (Wani 2016). NR (nicotinamide riboside, 3-aminocarbonyl-1-b-D-ribofuranosyl-pyridinium, Elysium Health, Inc., New York, NY) was dissolved in vaccination-grade water, and the pH of the solution neutralized (pH 7.0) before oral administration (185 mg/kg × day, Obrador 2020). FSL-1 (InvivoGen, San Diego, CA) was resuspended in sterile vaccination-grade water, and administered IP (0.25 mg/kg × day, the volume administered was of 50-60 mL depending on the weight of the mouse, as recommended by Kurkjian 2017). Pharmaceutical grade amifostine (Ethyol) was obtained from Cumberland Pharmaceuticals (Nashville, TN) as 500 mg sterile lyophilized powder vial, and reconstituted with physiological saline prior to use.

### Pathology

Mice were removed from the study either dead, moribund, or scheduled for sacrifice. All mice were necropsied, and approx. 30 tissues or organs as well as all gross lesions were collected for microscopic examination. After fixation in 10% neutral-buffered formalin, all tissues were processed routinely and stained with hematoxylin and eosin for histopathological evaluation.

Femurs were isolated post euthanasia of mice 14 days after the irradiation. Bone marrow cells were flushed from isolated femurs using 26-gauge needle with pre-chilled phosphate-buffered saline. Single cell suspension was prepared by gentle pipetting of the flushed cells few times and number of viable bone marrow nucleated cells (BMNCs) was counted using a hemocytometer (Neubauer, Marienfeld, Germany) and expressed as ×10⁶/femur.

### Rotarod test

Functions of the test include evaluating balance, grip strength and motor coordination of the subjects; especially in testing the effect of experimental drugs. For this test (Rota Rod, Harvard Apparatus, Holliston, MA) each animal was given three trials and the maximum period (seconds) that it could remain on a rotating axle (3.5 cm diameter; speed of rotation: 15 rpm) without falling was measured. Each mouse was given up to three attempts for an arbitrary limit of 1200 seconds and the longest period was recorded. Changes over time in the different groups were monitored weekly.

### Pterostilbene levels

Liquid chromatography and mass spectrometry (LC-MS/ MS) was performed, as previously described (Ferrer 2005), using a TSQ Vantage^{™} Triple Quadrupole Mass Spectrometer (Thermo Fisher Scientific) equipped with a Shimadzu LC-10ADvp. pump and an SLC-10Avp. controller system with an SIL-10ADvp. autoinjector.

### Silibinin levels

Measurement was based on the methodology described by Song et al. (2019). Briefly, samples were analyzed through HPLC (Ultimate 3000, Thermo Fisher Scientific) and MS/MS (Waters TQ detector triple quadrupole) systems on a Hypurity^{™} C18 column (50 × 4.6 mm) using an injection volume of 5 µL. The mobile phases consisted of water (A) and acetonitrile (B). The gradient elution system was optimized as follows: 0-3.0 min 20% - 100% B, 3.0-5.0 min 100% - 100% B, 5.0-5.5 min 100% - 20% B, 5.5-7.5 min 20% B. The flow rate was 0.5 mL/min. MS/MS data acquisition was performed under negative electrospray ionization mode. The multiple reaction monitoring mode was employed to monitor SIL with the precursor-to-product ion transition of m/z 481.3/125.0. The fragmentary voltage was 150 V, and the collision energy 15 eV. Tissue sample preparation was as described for determination of PTER levels (above).

### Blood NAD⁺ levels

Measurement of NAD⁺ levels in total blood was performed by HPLC following the methodology described by Yoshino and Imai (Yoshino et al. 2013) For this procedure we used an HPLC system (Shimadzu, Kyoto, Japan) with a Supelco LC-18-T column (15 cm × 4.6 cm; Sigma Aldrich) and a Hypercarb column (15 cm × 4.6 cm; Thermo Fisher Scientific, Waltham, MA), respectively. Whole-blood samples (0.5 ml) were collected in sodium citrate tubes at 4°C. Then a whole-blood aliquot of 0.1 ml was transferred to a cryovial containing 0.9 ml of 0.5 M ice-cold perchloric acid to lyse all blood cells. Samples were centrifuged at 10,000 g × 10 min (4°C). Supernatants were stored at -80°C until analyzed.

### Evaluation of therapy-induced in vivo toxicity

This included blood cell count and chemistry based on NIH standard methodology.

### Chromosomal aberrations and double-strand DNA breaks

We have followed the technique described by M'kacher et al. (2015) where telomere and centromere staining facilitates the easy detection of dicentrics, centric rings, and acentric chromosomes of premature chromosome condensation in lymphocytes using optimized hybridization conditions and fusion capture of interphase chromosomes. This approach presents a major advantage since damage can be observed within hours after blood sampling. Blood was collected 8 h after irradiation as suggested in the procedure (M'kacher 2015), thus allowing time for DNA repair and the occurrence of dicentrics, centric rings, and acentric chromosomes

Peripheral blood lymphocytes were isolated as described here below. Premature chromosome condensation (PCC) was performed as previously described (Pantelias 1983) using CHO cells (ATCC). The condensed human chromosomes were distinguished from the CHO chromosomes according to their morphologic characteristics (M'kacher 2015). This procedure required approx. 4 h.

Telomeres and centromeres of PCC fusions were stained using the Q-FISH technique with a Cy-3-labeled peptide nucleic acid (PNA) probe specific for TTAGGG of telomere and a fluoroscien isothiocyanate-labeled PNA probe specific for centromere sequences (M'kacher 2015) (both from ThermoFisher Scientific, Waltham. MA). Telomere and centromere-stained fusions were automatically recorded using the Autocapt software from Metasystems (Altlussheim, Germany) and a high resolution CCD camera (C91002-23B, Hamamatsu Photonics, Herrsching, Germany).

In the same slide two different manual procedures were performed as described by M'kacher et al. (2015). Briefly, first using the reverse 4',6-diamidino-2-phenylindole, and scoring rings and excess acentric chromosomes. Second using the telomeres and centromeres staining to score dicentrics, centric rings, and acentric chromosomes from interstitial deletions without telomere staining, acentric chromosomes with only 1 telomere, representing terminal deletions, and acentric chromosomes with 2 telomeres derived from the fusion of 2 acentric chromosomes accompanying the formation of dicentrics or acentric rings. Resulting double-strand DNA breaks (DSBs) were calculated (M'kacher 2015).

### Micronuclei formation

For this purpose the femoral bone marrow was collected from sacrificed mice, and smear was prepared and fixed using methanol. After drying, double staining was performed using May Grunwald and Giemsa stains (Schmid 1975). According to this method, polychromatic erythrocytes (PCE) stained reddish-blue and normochromatic erythrocytes (NCE) stained orange, while nuclear materials were dark purple. The micronucleated polychromatic (MnPCE) and normochromatic (MnNCE) cells were counted out of 2000 cell under oil immersion.

### Isolation and incubation of lymphocytes, hepatocytes, and intestinal epithelial cells

Isolation of hepatocytes was performed according to previously reported methodology (Berry 1969). Blood mononuclear cells were obtained from heparinized blood by Accuspin-Histopaque (Sigma-Aldrich, St. Louis, MO) gradient centrifugation. Mononuclear cells were washed twice and resuspended in Krebs-Henseleit bicarbonate medium (pH 7.4) with 0.3% bovine serum albumin at a concentration of 20 × 10⁶ cells/ml. Further positive selection of lymphocyte subsets by monodispersed immunomagnetic Dynabeads (Dynal) was performed at 4°C, as described elsewhere (Thornton 2003). Hepatocyte and lymphocyte cell volumes were obtained as previously described (Estrela 1992).

Isolated hepatocytes were incubated in flasks (about 10 mg dry wt/ml) at 37°C in Krebs-Henseleit bicarbonate medium (KHBM, pH 7.4) containing 1.3mM-CaCl₂, 5%fat-free bovine serum albumin, and in the presence of glucose (5mM). The gas atmosphere was O₂/CO₂ (19:1). Isolated lymphocytes were incubated (10⁶ cells/flask) at 37°C in Krebs-Ringer medium with 5% fat-free bovine serum albumin and in the presence of glucose (5mM) and glutamine (2 mM). After incubation, the cells were disrupted by the addition of 0.2 ml of 25% perchloric acid. Protein was removed by centrifugation and the supernatant was neutralized with 20 ml of a 40% KOH solution and a Tris-(hydroxymethyl) aminomethane/KOH (0.5-2.0 M) solution for the measurement of the metabolites.

Primary intestinal epithelial cells (IECs) were isolated using a protocol adapted from Graves et al. (2014). Colon tissue was prepared by removing the longitudinal muscle layer and washing with ice-cold Mg²⁺- and Ca²⁺-free Hank's Balanced Salt Solution (HBSS) containing 100 U penicillin, 100 µg/ml streptomycin, 25 µg/ml gentamycin and 0.5 mM dithiothreitol (DTT). Tissue was cut into small pieces, suspended in 50 ml HBSS wash solution and shaken, and the contents were allowed to settle for 2 min. The supernatant was removed and the settled contents were washed 3 times. The settled contents were minced and suspended in HBSS. The resulting suspension was passed over a 1000 µm² mesh filter. Remaining tissue was digested in a buffer containing 75 U/ml collagenase type XI (Sigma-Aldrich), 25 µg/ml dispase neutral protease II (ThermoFisher Scientific, Waltham, MA), 0.5 mM DTT, and 2% v/v fetal bovine serum (FBS) (ThermoFisher Scientific) in Dulbecco's Modification of Eagles Medium (DMEM) (Sigma-Aldrich). The digestion buffer containing the tissue was then placed in a 37 °C incubator and allowed to shake at 200 rpm for 2 h. The resulting digestion mixture was again passed over a 1000 µm2 filter, and the tissue fragments atop the filter were washed with 25 ml complete growth media (DMEM, 8.5 g/l sodium pyruvate, 2% v/v FBS, 0.25 U/ml insulin, 100 U penicillin, 100 µg/ml streptomycin, 25 µg/ml gentamycin, 5 µg/ml transferrin, and 10 ng/ml EGF containing 2% w/v D-sorbitol. Tissue debris remaining in the filter was discarded, and the effluent containing proliferative crypt structures was centrifuged at 200g, for 5 min, at 4 °C. The remaining pellet containing isolated intestinal crypts was suspended in DMEM. The crypts were suspended in the complete growth media, plated at a density of approximately 2000 crypts/ml/well in a 12-well type I collagen-coated culture dish (ThermoFisher Scientific) and incubated at 37 °C, O₂/CO₂ (19:1).

Rates of glucose and glutamine utilization were measured as previously described (Newsholme 1987).

### Cell culture

Isolated hepatocytes were cultured in William's complete medium at a concentration of 10⁶ cells/mL. Isolated C2D⁺ lymphocytes were cultured in PB-MAX medium (GIBCO, ThermoFisher Scientific) at a concentration of 2 × 10⁶ cells/mL. IECs (10⁶ cells/mL) were cultured in DMEM supplemented with 10% heat-inactivated FBS, and 300 mg/ml L-glutamine. All cells were cultured at 37°C in a humidified atmosphere of 95% air and 5% CO₂.

### DNA oxidation

Tissue samples were homogenized in 10 mM Tris (pH 7.0) +1 mM EDTA + 150 mM NaCl. Thereafter they were incubated first with RNase A at 37 °C for 30 min and then with proteinase K at 55 °C overnight. This was followed by chloroform/isoamyl alcohol extraction. Samples of 100µg of isolated DNA were digested to nucleosides with nuclease P1 and alkaline phosphatase (Crain 1990). Analysis of modified DNA base 8-hydroxy-2'-deoxyguanosine (8OHdG) was accomplished by UPLC-MS/MS.

### Lipid peroxidation

For isoprostane determination, tissue samples were homogenized in 0.1M phosphate buffer (pH 7.4) + 1mM EDTA + 0.005% butylated hydroxytoluene. Isoprostanes were measured using the 8-isoprostane EIA kit (Cayman Chemical Co.) and following the manufacturer's protocol.

### Protein carbonylation

Carbonylation of proteins, an irreversible oxidative damage, was measured using the Protein Carbonyl Fluorometric Assay Kit (Cayman Chemical Co.) and following the manufacturer's protocol.

### Enzyme activities and metabolite levels

Isolated cells were homogenized in 0.1M phosphate buffer (pH 7.2) at 4 °C. γ-glu tamylcysteine ligase (GCL), glutathione peroxidase (GPX), catalase (CAT), and superoxide dismutase 1 and 2 (SOD1 and SOD2) activities were measured as previously described in detail (Benlloch 2016). Protein concentration was determined with the Pierce BCA protein assay (Thermo Fisher Scientific). Glutathione (□-L-glutamyl-L-cystenyl-glycine, GSH) was determined by LC/MS as previously reported (Obrador 2014). Cell processing was performed according to published methodology, where rapid N-ethylmaleimide derivatization was used to prevent GSH auto-oxidation (Asensi 1994). NAD⁺ and NADH in isolated cells were quantitated using assay kits from MyBiosource (San Diego, CA).

### RT-PCR and detection of mRNA

Total RNA was isolated using the TRIzol kit from Invitrogen (San Diego, CA) following the manufacturer's instructions. cDNA was obtained using a random hexamer primer and a MultiScribe Reverse Transcriptase kit as recommended by the manufacturer (Taq-Man RT Reagents; Applied Biosystems, Foster City, CA). PCR master mix and AmpliTaq Gold DNA polymerase (Applied Biosystems) were added to the primers previously reported (Benlloch 2016). Real-time quantification of mRNA relative to glyceraldehyde 3-phosphate dehydrogenase (GAPDH) was performed as previously reported (Benlloch 2016).

### Comet assay

This assay is a single cell gel electrophoresis assay for simple evaluation of cellular DNA damage. We used the assay kit from Abcam (ab238544) (Cambridge, UK). The manufacturer's instructions were followed step by step.

### Nicotinamide phosphoribosyltransferase activity assay

Formation of 14C-nicotinamide mononucleotide (NMN) from 14C-nicotinamide (NAM, (GE Healthcare Life Sciences, Björkgatan, Uppsala, Sweden) can be measured by precipitation of 14C-NMN in acetone on filter paper and scintillation counting. Cell dispersion was carried out by trypsinization (in Mg²⁺⁻ and Ca2⁺⁻free PBS supplemented with 0.2% trypsin, 0.5 mM EDTA, and 5 mM glucose for 3 min at 37 °C). Cell pellets were obtained by centrifugation (800 g × 5 min at 4 °C), and then 100 µl of phosphate buffer [0.01 M Na2HPO4/NaH2PO4 buffer, pH 7.4, containing protease inhibitor (Sigma- Aldrich)] was added to each cell sample. Samples were further processed, and NAMPT activity was determined on 30 µg of protein as described previously (Elliott GC, et al. 1980, and Schuster S, et al. 2014). Radioactivity (disintegrations/min) was measured using a Tri-Carb^{®} Liquid Scintillation Counter from Perkin-Elmer. Background disintegrations/min from scintillation fluid was subtracted from each measurement.

### Colony forming cells in the bone marrow

Mice were sacrificed under aseptic conditions and femurs were isolated on day 7 post irradiation. Bone marrow cells were flushed from the isolated femurs (see above) with phosphate-buffered saline (4 °C) supplemented with 5% fetal bovine serum. Single-cell suspension was passed through 100 µm nylon mesh strainer to remove debris and clumps. Cells were washed twice with phosphate-buffered saline (4 °C) and counted using a hemocytometer. Cells were plated ex vivo at a concentration of 5 × 10⁴ cells per 35 mm cell culture dish in Methocult GF M3434 (Stemcell Technologies, Vancouver, Canada) growth medium according to the manufacturer's protocol and incubated for 14 days at 37 °C, 5% CO₂, 95% humidity. Colonies were visualized on day 14 of culture using light microscopy (AxioCam MRc5, Zeiss, Oberkochen, Germany) under a polarised light and scanned in a 60 mm gridded scoring Petri dish (Stemcell Technologies). Hematopoietic colony forming cells CFU-GM and CFU-GEMM were quantified.

### Preparation of cell, nuclear, and mitochondrial extracts

Cell extracts were prepared using the Whole Cell Extraction Kit of Sigma Aldrich. The NE-PER^{™} Nuclear and Cytoplasmic Extraction kit (ThermoFisher Scientific) enables stepwise separation and preparation of cytoplasmic and nuclear extracts from mammalian cells or tissues. Mitochondrial extracts were prepared following the Cell Fractionation and Organelle Isolation procedures standardized by ThermoFisher Scientific.

### Gene silencing

The PSilencer 3.1-H1 linear vector from Ambion Inc. (Austin, TX) was used to obtain long term gene silencing. To target mouse Nfkbie, a siRNA Oligo Duplex (Locus ID 18037) from Origen (Rockville, MD) was used. The siRNAs to target mouse Nfe2l2 (RefSeq NM_010902.3), Ppargc1 (RefSeq NM_008904.2), Sirt1 (RefSeq NM_019812.2), Sirt3 (RefSeq MN_022433.2), Parp1 (RefSeq NM_007415.2), SOD2 (RefSeq NM_013671.3), and GPX1 (RefSeq NM_008160.6) were from Invitrogen. In all cases silencing procedures followed the technical recommendations of the manufacturers. Control experiments were performed using equivalent amounts of the corresponding sense oligonucleotides and scrambled oligonucleotides with the same base composition and a randomized sequence. Silencing was confirmed by immunoblotting. Invitrogen Lipofectamine RNAiMAX Transfection Reagent and manufacturer's protocol were used for delivery of siRNA.

### Cell death analysis

Apoptotic and necrotic cell death were distinguished by using fluorescence microscopy. For this purpose, isolated MNs were incubated with Hoescht 33342 (10 mM; which stains all nuclei) and propidium iodide (10 mM; which stains nuclei of cells with a disrupted plasma membrane), for 3 min, and analyzed using a Diaphot 300 fluorescence microscope (Nikon, Tokyo, Japan) with excitation at 360 nm. Nuclei of viable, necrotic, and apoptotic cells were observed as blue round nuclei, pink round nuclei, and fragmented blue or pink nuclei, respectively. About 1,000 cells were counted each time.

### Western blots

Western blot analysis was performed as previously described (Benlloch M, et al. 2016). Proteins were transferred to a nitrocellulose membrane and subjected to western blotting with specific anti-human monoclonal antibodies (OriGene, Rockville, MD; and Abcam). Blots were developed using horseradish peroxidase-conjugated secondary antibody and enhanced chemiluminescence (ECL system; GE HealthCare Life Sciences). Protein bands were quantified using laser densitometry.

### Tumor xenografts

Human A549 and MDA-MB-231 cells were from the ATCC (Manassas, VA). Cells were grown in DMEM (Invitrogen, San Diego, CA), pH 7.4, supplemented with 10% heat-inactivated FCS (Biochrom KG, Berlin, Germany), 100 units/mL penicillin and 100 µg/mL streptomycin. Cells were plated (20,000 cells/cm²) and cultured at 37°C in a humidified atmosphere with 5% CO₂. Cells were harvested by incubation for 5 min with 0.05% (w/v) trypsin (Sigma Aldrich, St. Louis, MO) in PBS, pH 7.4, containing 0.3 mM EDTA, followed by the addition of 10% FCS to inactivate the trypsin. Cells were allowed to attach for 12 h before any treatment addition. Cell number and viability were determined using a BioRad (Hercules, CA) TC20 Automated Cell Counter. Nude (nu/nu) mice (male, 9-10 weeks old, Charles River Laboratories, Wilmington, MA) were fed ad libitum on a standard diet (Letica, Rochester Hills, MI), and kept on a 12-h-light/12-h-dark cycle with the room temperature at 22°C. Procedures were in compliance with international laws and policies (EEC Directive 86/609, OJ L 358. 1, December 12, 1987; and NIH Guide for the Care and Use of Laboratory Animals, NIH Publ. No. 85-23, 1985). Tumor cells were harvested from culture flasks by exposure to 0.02% EDTA (5 min at 37 °C), washed twice in DMEM, resuspended in the same culture medium, and injected s.c. on the back of the animal (5 × 10⁶ cells/mouse). Local tumor growth was determined using calipers every 2 days, starting on the 2nd day of treatment.

### Statistics

Data are presented as mean values ± SD for the number of different experiments. Statistical analyses were performed using Student's t-test, and *P* < 0.05 was considered significant.

The G*Power 3.1.9.2 software was used to determine the sample size (ANOVA test, effect size = 0.21, α = 0.05, β =0.80 and the indicated groups). Survival data were analyzed with Kaplan- Meier curves and LogRank (Mantel-Cox) test.

### References

Asensi, M. et al. A high-performance liquid chromatography method for measurement of oxidized glutathione in biological samples. Anal Biochem 217, 323-328 (1994).
Benlloch M, et al. Pterostilbene Decreases the Antioxidant Defenses of Aggressive Cancer Cells In Vivo: A Physiological Glucocorticoids- and Nrf2-Dependent Mechanism. Antioxid Redox Signal. 2016;24:974-90
Berry, M. N., and Friend, D. S. (1969) High-yield preparation of isolated rat liver parenchymal cells: a biochemical and fine structural study. J Cell Biol. 43, 506-520.
Benlloch, M., Obrador, E., Valles, S. L., Rodriguez, M. L., Sirerol, J. A., Alcácer, J., Pellicer, J. A., Salvador, R., Cerdá, C., Sáez, G. T., and Estrela, J. M. (2016) Pterostilbene Decreases the Antioxidant Defenses of Aggressive Cancer Cells In Vivo: A Physiological Glucocorticoids- and Nrf2-Dependent Mechanism. Antioxid. Redox Signal. 24, 974-990.
Boehning, D., Sedaghat, L., Sedlak, T. W. & Snyder, S. H. Heme Oxygenase-2 Is Activated by Calcium-Calmodulin. J. Biol. Chem. 279, 30927-30930 (2004).
Cantó C, Sauve AA, Bai P. Crosstalk between poly(ADP-ribose) polymerase and sirtuin enzymes. Mol Aspects Med. 2013 Dec;34(6):1168-201.
Crain, P. F. Preparation and enzymatic hydrolysis of DNA and RNA for mass spectrometry. Methods Enzymol. 193:782-790; 1990.
Elliott GC, et al. A rapid procedure for assaying nicotinamide phosphoribosyltransferase. Anal Biochem. 1980;107:199-205;
Estrela JM, Hernandez R, Terradez P, Asensi M, Puertes IR, and Vina J. Regulation of glutathione metabolism in Ehrlich ascites tumour cells. Biochem J 286 (Pt 1): 257-262, 1992.
Estrela JM, Mena S, Obrador E, Benlloch M, Castellano G, Salvador R, Dellinger RW. Polyphenolic Phytochemicals in Cancer Prevention and Therapy: Bioavailability versus Bioefficacy. J Med Chem. 2017 Dec 14;60(23):9413-9436.
Ferrer P, Asensi M, Segarra R, Ortega A, Benlloch M, Obrador E, Varea MT, Asensio G, Jorda L, and Estrela JM. Association between PTERostilbene and quercetin inhibits metastatic activity of B16 melanoma. Neoplasia 7: 37-47, 2005.
Fischer N, Seo EJ, Efferth T. Prevention from radiation damage by natural products. Phytomedicine. 2018 Aug 1;47:192-200.
Graves CL, Harden SW, LaPato M, Nelson M, Amador B, Sorenson H, Frazier CJ, Wallet SM. A method for high purity intestinal epithelial cell culture from adult human and murine tissues for the investigation of innate immune function. J Immunol Methods. 2014 Dec 1;414:20-31.
Groen, A. K., Sips, H. J., Vervoorn, R. C., and Tager, J. M. (1982) Eur. J. Biochem. 122, 87-93.
Klimberg VS, Souba WW, Dolson DJ, Salloum RM, Hautamaki RD, Plumley DA, Mendenhall WM, Bova FJ, Khan SR, Hackett RL, Bland KI, Copeland III EM. Prophylactic glutamine protects the intestinal mucosa from radiation injury. Cancer. 1990 Jul 1;66(1):62-8.
Kurkjian CJ, Guo H, Montgomery ND, Cheng N, Yuan H, Merrill JR, Sempowski GD, Brickey WJ, Ting JP. The Toll-Like Receptor 2/6 Agonist, FSL-1 Lipopeptide, Therapeutically Mitigates Acute Radiation Syndrome. Sci Rep. 2017 Dec 11;7(1):17355.
Martens CR, Denman BA, Mazzo MR, Armstrong ML, Reisdorph N, McQueen MB, Chonchol M, Seals DR. Chronic nicotinamide riboside supplementation is well-tolerated and elevates NAD+ in healthy middle-aged and older adults. Nat Commun. 2018 Mar 29;9(1):1286.
Mavragani IV, Laskaratou DA, Frey B, Candéias SM, Gaipl US, Lumniczky K, Georgakilas AG. Key mechanisms involved in ionizing radiation-induced systemic effects. A current review.
Toxicol Res (Camb). 2015 Aug 11;5(1):12-33.
M'kacher R, El Maalouf E, Terzoudi G, Ricoul M, Heidingsfelder L, Karachristou I, Laplagne E, Hempel WM, Colicchio B, Dieterlen A, Pantelias G, Sabatier L. Detection and automated scoring of dicentric chromosomes in nonstimulated lymphocyte prematurely condensed chromosomes after telomere and centromere staining. Int J Radiat Oncol Biol Phys. 2015 Mar 1;91(3):640-9.
Newsholme, P., Gordon, S., and Newsholme, E. A. (1987) Rates of utilization and fates of glucose, glutamine, pyruvate, fatty acids and ketone bodies by mouse macrophages. Biochem. J. 242, 631-636.
Obrador, E., Valles, S. L., Benlloch, M., Sirerol, J. A., Pellicer, J. A., Alcácer, J., Coronado, J. A.-F., and Estrela, J. M. (2014) Glucocorticoid receptor knockdown decreases the antioxidant protection of B16 melanoma cells: an endocrine system-related mechanism that compromises metastatic cell resistance to vascular endothelium-induced tumor cytotoxicity. PloS One. 9, e96466.
Obrador E, Salvador R, Marchio P, López-Blanch R, Jihad-Jebbar A, Rivera P, Vallés SL, Banacloche S, Alcácer J, Colomer N, Coronado JA, Alandes S, Drehmer E, Benlloch M, Estrela JM. Nicotinamide Riboside and Pterostilbene Cooperatively Delay Motor Neuron Failure in ALS SOD1G93A Mice. Mol Neurobiol. 2020 Nov 10. doi: 10.1007/s12035-020-02188-7.
Pantelias GE, Maillie HD. A simple method for premature chromosome condensation induction in primary human and rodent cells using polyethylene glycol. Somat Cell Genet 1983;9:533-547.
Patyar RR, Patyar S. Role of drugs in the prevention and amelioration of radiation induced toxic effects. Eur J Pharmacol. 2018 Jan 15;819:207-216.
Riklis E, Kol R, Marko R. Trends and developments in radioprotection: the effect of nicotinamide on DNA repair. Int J Radiat Biol. 1990 Apr;57(4):699-708.
Qi Z, Whitt I, Mehta A, Jin J, Zhao M, Harris RC, et al. Serial determination of glomerular filtration rate in conscious mice using FITC-inulin clearance. Am J Physiol Renal Physiol. 2004;286:F590-596.
Reisz JA, Bansal N, Qian J, Zhao W, Furdui CM. Effects of ionizing radiation on biological molecules-mechanisms of damage and emerging methods of detection. Antioxid Redox Signal 2014, 21: 260-92.
Riley, P.A., 1994. Free radicals in biology: oxidative stress and the effects of ionizing radiation. Int. J. Radiat. Biol. 65, 27-33.
Schmid W. 1975. The micronucleus test. Mutat Res Mutagen Relat Subj. 31: 9-15.
Sirerol JA, Feddi F, Mena S, Rodriguez ML, Sirera P, Aupí M, Pérez S, Asensi M, Ortega A, Estrela JM. Topical treatment with pterostilbene, a natural phytoalexin, effectively protects hairless mice against UVB radiation-induced skin damage and carcinogenesis. Free Radic Biol Med. 2015 Aug;85:1-11.
Song Y, Sun H, Xiao J, Wang F, Ding Y, Zhao J, Bai J, Cheng L, Gao K, Liu M, Guo Q, Zhang Y, Gao W, Jia Y, Wen A. Development of a liquid chromatography-tandem mass spectrometric (LC-MS/MS) method for simultaneous determination of epigallocatechin-3-gallate, silibinin, and curcumin in plasma and different tissues after oral dosing of Protandim in rats and its application in pharmacokinetic and tissue distribution studies. J Pharm Biomed Anal. 2019 Jun 5;170:54-62.
Schuster S, et al. Resveratrol differentially regulates NAMPT and SIRT1 in Hepatocarcinoma cells and 833 primary human hepatocytes. PLoS One. 2014;9:e91045
Thornton, A. M. (2003) Fractionation of T and B cells using magnetic beads. Curr. Protoc. Immunol. ChaPTER 3, Unit 3.5A.
Wani TA, Shah AG, Wani SM, Wani IA, Masoodi FA, Nissar N, Shagoo MA. Suitability of Different Food Grade Materials for the Encapsulation of Some Functional Foods Well Reported for Their Advantages and Susceptibility. Crit Rev Food Sci Nutr. 2016 Nov 17;56(15):2431-2454
Williams JP, Brown SL, Georges GE, Hauer-Jensen M, Hill RP, Huser AK, Kirsch DG, Macvittie TJ, Mason KA, Medhora MM, Moulder JE, Okunieff P, Otterson MF, Robbins ME, Smathers JB, McBride WH. Animal models for medical countermeasures to radiation exposure. Radiat Res. 2010 Apr;173(4):557-78.
Yoshino J, Imai S-I. Accurate measurement of nicotinamide adenine dinucleotide (NAD+) with high-performance liquid chromatography. Methods Mol Biol. 2013; 1077: 203-215.

### EXAMPLE 2: PT and radioprotective effect

First, the effects of γ radiation and mice survival were studied. Mice received whole-body γ radiation were studied (Figure 1A). Mice received whole-body γ radiation (one single dose; ¹³⁷Cs, 0.5-0.6 Gy/min; Biobeam 8000, GSM GmbH). One Gy= absorption of 1 J of energy/kg of matter. LD50/30 of approx. 7.2 Gy (n=20 per radiation dose).

Next, the thirty-days survival of mice treated with γ rays (LD50/30) and pterostilbene (PT) was analysed (Figure 1B). For that, Swiss albino mice (Charles River, n=20/group) were treated with amifostine (2-(3-aminopropylamino) ethylsulfanylphosphonic acid) (dissolved in physiological saline; one single oral dose 30 min before irradiation; recommended 200 mg/m² in humans was adapted to mice following FDA's guidelines) or pterostilbene (dissolved in 2-hydroxypropyl-b-cyclodextrin, then suspended in 0.3% carboxymethylcellulose; adm. orally, once per day, 5 days before and 2 days after irradiation). Mice received whole-body γ radiation (one single dose of 7.2 Gy of ¹³⁷Cs at 0.5-0.6 Gy/min, equipment= Biobeam 8000, GSM GmbH). One Gy= absorption of 1 J of energy/kg of matter. Data were analyzed with LogRank (Mantel-Cox) test (*comparing all groups vs. controls treated with vehicle). The results in Figure 1B show that the best effect on improving survival (60 days) is exerted by the dose of 100 mg Pterostibene/kg.

Survival of mice treated with γ rays (LD50/30) and resveratrol (Resv) was also analysed and the results are shown in Figure 1C. Swiss albino mice (n= 20/group) were treated with Resvl (dissolved in 2-hydroxypropyl-b-cyclodextrin, then suspended in 0.3% carboxymethylcellulose; adm. orally, once per day, 5 days before and 2 days after irradiation). Mice received whole-body γ radiation (one single dose of 7.2 Gy of ¹³⁷Cs at 0.5-0.6 Gy/min, Mortazavi SMJ et al. Dose-Response 11: 281-292, 2013; equipment= Biobeam 8000, GSM GmbH). One Gy= absorption of 1 J of energy/kg of matter. The results showed that the radioprotective effects of Resv were lower than those of PT.

Plasma and extravascular levels of pterostilbene after its oral administration to mice were studied. Mice were treated with 200 mg Pter/kg b.w. A group of 4-5 animals was sacrificed per each time point. Pter was dissolved in 2-hydroxypropyl-b-cyclodextrin, then suspended in 0.3% carboxymethylcellulose. Nondetectable= n.d. *P< 0.1 comparing data obtained at 10-1440 min versus those found at 5 min. The results are shown in Table 1 below:

| | **Pter (µM)** | | | | |
|---|---|---|---|---|---|
| **Time (min)** | **Plasma** | **Brain** | **Lung** | **Liver** | **Kidney** |
| 5 | 5 ± 2 | 2 ± 1 | 7 ± 2 | 3 ± 1 | 1 ± 0.5 |
| 10 | 18 ± 4* | 6 ± 2* | 15 ± 6 | 8 ± 3* | 4 ± 2* |
| 30 | 55 ± 9* | 10 ± 3* | 23 ± 5* | 11 ± 3* | 6 ± 3* |
| 60 | 98 ± 17* | 24 ± 7* | 35 ± 8* | 17 ± 5* | 10 ± 4* |
| 120 | 46 ± 12* | 14 ± 4* | 21 ± 4* | 12 ± 4* | 8 ± 2* |
| 240 | 21 + 6* | 8 ± 3* | 12 ± 3* | 7 ± 2* | 2 ± 0.5 |
| 480 | 14 ± 5* | 3 ± 1 | 2 ± 1 | 1 ± 0.5 | n.d. |
| 720 | 1 ± 0.5 | n.d. | n.d. | n.d. | n.d. |
| 1440 | n.d. | n.d. | n.d. | n.d. | n.d. |

These results show that Pterostilbene levels peak, in all tissues studied, at 60 min after administration.

Altogether, the results above confirm the potential of PTER as radioprotector.

### EXAMPLE 3: Combination of PT + SIL

In order to elucidate if pterostilbene could be combined with other polyphenols to achieve a better radioprotective effect, Swiss albino mice (n= 20/group) were treated with pterostilbene and/or other polyphenols. For oral administration PT was solubilized in 2-hydroxypropyl-b-cyclodextrin, then suspended in 0.3% carboxymethylcellulose. All other polyphenolic phytochemicals assayed represent main flavonoids and non-flavonoids, i.e. gallic acid (a phenolic acid), caffeic acid (a hydroxycinnamic acid), curcumin (a curcuminoid), epigallocatechin gallate (a flavanol), genistein (a isoflavone), quercetin (a flavonol), luteolin (a flavone), naringenin (a flavanone), delphindin (an anthocyanidin), and phloridzin (a chalcone), and were solubilized as PT before oral administration. Doses for each phytochemical were selected from the max non-toxic doses published. Data were analyzed with LogRank (Mantel-Cox) test (*comparing all groups vs. controls treated with vehicle, +comparing all groups vs. the group treated with PT alone) (Figure 2).

The results showed that the combination of PT with the different polyphenols resulted in different effects:
a) In some cases, the combination resulted in lower survival than the survival provided the PT administrated separately. This is seen, for instance, in the combination of PT with Gallic acid at 60 days post-irradiation.
b) In some cases, the survival resulted from the combination of the two compounds was similar to that provided by PT administered alone, indicating that the addition of the other compound barely influenced the overall survival. This is seen, for instance, in the combination of PT with Delphinidin at 60 days post-irradiation.
c) In some other cases, the resulting survival equals to approximately the sum of the survivals exerted by the two compounds administered individually. This is seen, for instance, in the combination of PT with Gesnistein at 60 days post-irradiation
d) Lastly, and most importantly, the resulting survival of the combination of the two compounds resulted in a synergistic effect that produced an exceptionally increased survival which cannot be derived from the sum of the effects of the two compounds administered separately. This last case only happened with the combination of Silibinin (SIL) and PT, and happened in both at 30 and 60 days post-irradiation.

Altogether, these results show that the combination of SIL+PT exerts a synergistic radioprotective effect achieving high percentage of survival 30 and 60 days after γ radiation.

In view of this results, the enhanced survival and enhanced radioprotection at longer period of times was studied. Swiss albino mice (n= 20/group) were treated IP with PT (100 mg/kg) and SIL (70 mg/kg) x 7 days (5 days before and 2 days after γ irradiation). For IP administration, a PT salt (pterostilbene phosphate disodium salt, SYNCOM, Groningen, The Netherlands) and SIL salt (silibinin-C-2',3-dihydrogen succinate, disodium salt, Rottapharm/Madaus, Cologne, Germany) were dissolved in sterile vaccination-grade water. Figure 3A shows the Kaplan-Meier curves and LogRank (Mantel-Cox) test (*comparing the PT+SIL-treated group vs. controls treated with vehicle). The IP administration was selected to facilitate the regular administration to a high number of mice. As can be seen in Figure 3A, the combination of PT+SIL provided long term protection, with around 25% (5/20) survival after 1 year post-irradiation time. These results confirm the synergistic enhanced effect of these two compounds when administered in combination and demonstrate that their combined radioprotective effect lasts for long period of times.

Additionally, Figure 3B shows a comparison between administration of PT + SIL before or after the γ irradiation: Swiss albino mice (n= 20/group) were treated IP with PT (100 mg/kg) and SIL (70 mg/kg) x 7 days (5 days before and 2 days after a LD50/30 of γ irradiation, light grey bars; or 7 days after γ irradiation, dark grey bars). Data were analyzed with LogRank (Mantel-Cox) test (*comparing the group treated after irradiation vs. that treated before irradiation). These results show that survival is higher if polyphenols are administered before the radiation.

Next, the effect of pterostilbene (PT) and silibinin (SIL) on the survival of γ irradiated (LD50/30) mice and the effect on body weight: Swiss albino mice (n= 20/group) were treated IP with PT (100 mg/kg) and SIL (70 mg/kg) x 7 days (5 days before and 2 days after γ irradiation). Statistical analyses were performed using Student's t-test (*P< 0.01 comparing PT+SIL vs. vehicle-treated controls). The results are shown in Figure 4A where it can be appreciated that polyphenols reduce the radiation-induced loss in body weight.

The effect of the combined administration of pterostilbene (PT) and silibinin (SIL) on the whole-body radiation-induced alteration of neuromotor functions in mice was also studied: Swiss albino mice (n= 20/group) were treated IP with PT (100 mg/kg) and SIL (70 mg/kg) x 7 days (5 days before and 2 days after γ irradiation). To study the neuromotor function, the standardized Rotarod test was performed as follows: Functions of the test include evaluating balance, grip strength and motor coordination of the subjects; especially in testing the effect of experimental drugs. For this test (Rota Rod, Harvard Apparatus, Holliston, MA) each animal was given three trials and the maximum period (seconds) that it could remain on a rotating axle (3.5 cm diameter; speed of rotation: 15 rpm) without falling was measured. Each mouse was given up to three attempts for an arbitrary limit of 1200 seconds and the longest period was recorded. Changes over time in the different groups were monitored weekly. As shown in Figure 4B, the results showed that neuromotor functions were not significantly altered either by the radiation and/or the administration of PT and SIL.

Next, expression analysis of different antioxidant defense-related enzyme activities in isolated cells were studied by RT-PCR. Statistical analyses were performed using Student's t-test (P< 0.01; *comparing PT+SIL+γ rays-treated mice vs. mice treated with γ rays alone; ⁺comparing PT+SIL-treated mice vs. controls treated with vehicle). These results are shown in Figure 5, and it was concluded that radiation decreases the expression of all the antioxidant defense-related molecules studied, whereas PT and SIL counteract the radiation-induced effects.

Further, the protective effect of PT and SIL on the γ radiation induced oxidative damage was studying by evaluating different molecular markers. To do so, PT, SIL and γ rays were administered as and at the doses previously indicated (n=5 per molecule, cell type, and treatment), and statistical analyses were performed using Student's t-test (P< 0.01; *comparing all groups vs. controls; +comparing comparing PT+SIL+γ rays-treated mice vs. mice treated with γ rays alone). As shown in Figure 6, PT and SIL counteract the radiation-induced increase in different oxidative stress-related biomarkers.

In order to provide a complete analysis of the radioprotective effects of PT and SIL when administered in combination, the antioxidant defence mediated by GCL and GSH was also measured. Thus, PT, SIL and γ rays were administered as and at the doses previously indicated (n=5 per molecule, cell type, and treatment), and statistical analyses were performed using Student's t-test (P< 0.01; *comparing all groups vs. controls; +comparing comparing PT+SIL+γ rays-treated mice vs. mice treated with γ rays alone). As shown in Figure 7, PT and SIL counteract the radiation-induced decrease in different glutathione-related biomarkers.

In Figure 8, catalase and glutathione peroxidase (Fig.8A and B) and superoxide dismutases levels (Fig. 8C and D) were measured when PT, SIL and γ rays were administered as and at the doses previously indicated (n=5 per molecule, cell type, and treatment). Statistical analyses were performed using Student's t-test (P< 0.01; *comparing all groups vs. controls; +comparing comparing PT+SIL+ γ rays-treated mice vs. mice treated with γ rays alone). The results showed that PT and SIL counteract the radiation-induced decrease in different antioxidant enzyme activities.

Lastly, the effect of PT and SIL on γ radiation-induced chromosomal aberrations and micronuclei formation in circulating CD2+ lymphocytes was studied. Telomeres and centromeres of premature chromosome condensation fusions were stained using the Q-FISH technique. The telomeres and centromeres staining was used to score dicentrics, centric rings, and acentric chromosomes from interstitial deletions without telomere staining, acentric chromosomes with only 1 telomere, representing terminal deletions, and acentric chromosomes with 2 telomeres derived from the fusion of 2 acentric chromosomes accompanying the formation of dicentrics or acentric rings. Resulting double-strand DNA breaks (DSBs) were calculated. In the bone marrow double staining was performed using May Grunwald and Giemsa stains. According to this method, polychromatic erythrocytes (PCE) stained reddish-blue and normochromatic erythrocytes (NCE) stained orange, while nuclear materials were dark purple. The micronucleated polychromatic (MnPCE) and normochromatic (MnNCE) cells were counted. Statistical analyses were performed using Student's t-test (P< 0.01; *comparing all groups vs. controls; +comparing PT+SIL+γ rays-treated mice vs. mice treated with γ rays alone). The results are shown in Table 2 below:

| | **Chromosomal Alterations** | | | | | **Micronuclei** | |
|---|---|---|---|---|---|---|---|
| | **No.dicentrics/ 10³ cells** | **No.rings/ 10³ cells** | **No.excess acentrics/ 10³ cells** | **dicentrics+rings/ cell** | **DSB/ 10³ cells** | **MnPCE/ 10³ PCE** | **MnNCE/ 10³ PCE** |
| **Control (no treated)** | **8.9 ± 1.1** | **0 ± 0** | **9.7 ± 1.1** | **0.0089 ± 0.0011** | **0 ± 0** | **4.9 ± 0.7** | **5.3 ± 0.8** |
| **γ-irradiated+vehicle** | **3317 ± 175*** | **597 ± 51*** | **2692 ± 117*** | **3.9 ± 0.2*** | **797 ± 53*** | **209 ± 25*** | **24 ± 4*** |
| **γ-irradiated+PT+SiL** | **1063 ± 130*⁺** | **118 ± 23*⁺** | **608 ± 58*⁺** | **1.2 ± 0.1*⁺** | **133 ± 19*⁺** | **33 ± 9*⁺** | **6 ± 1⁺** |

As can be seen in Table 2, PT and SIL decrease all the radiation-induced cytogenetic alterations, i.e. chromosomal alterations and formation of micronuclei.

### EXAMPLE 4: Combination of PT and SIL with radiomitigators

We next asked whether the combination of radioprotectors (molecules that can reduce the direct damage caused by radiation) and radiomitigators (those that minimize toxicity even after radiation has been delivered) could favour a longer survival. To study this, PT and SIL were combined with the NAD+ booster nicotinamide riboside (NR) and with the toll like receptor 2/6 agonist FSL-1 lipopeptide (FSL-1)

Firstly, the effects of both molecules alone and in combination to confirm their radiomitigation properties were studied. To do so, NR (nicotinamide riboside, 3-aminocarbonyl-1-b-D-ribofuranosyl-pyridinium, Elysium Health, Inc., New York, NY) was dissolved in vaccination-grade water, and the pH of the solution neutralized (pH 7.0) before oral administration (185 mg/kg x day). FSL-1 (fibroblast-stimulating lipopeptide 1, InvivoGen, San Diego, CA) was resuspended in sterile vaccination-grade water, and administered IP (0.25 mg/kg x day, the volume administered was of 50-60 mL depending on the weight of the mouse). NR was administered daily for 30 days, one single dose per day, and starting after the irradiation. FSL-1 was administered only once, 24 h after the irradiation. The total number of mice used was 20. The results are shown in Figure 9, where it is observed that the survival at 60 days post γ irradiation provided by each of these molecules administered alone is 0% for NR, 5% for FSL-1, and 10% for the combination of NR+FSL1.

Next, swiss albino mice (n= 6/group) were treated with PT, SIL, or NR as scheduled previously and at the doses indicated above and statistical analyses were performed using Student's t-test (P< 0.01; *comparing 120 min vs. 30 min; + comparing 2 vs. 0 days; #comparing 3 or 15 days vs. 0 days) and the results are shown in Figure 10. As can be seen in this figure, PT and SIL levels are higher 30 min after their administration, whereas NR-induced NAD+ levels where found similar at the two time points measured (30 and 120 min).

Next, the four compounds PT, SIL, NR and FSL-1 were administered in n=20 mice/group as and at the doses indicated above. Figure 11A shows the administration schedule followed. Figure 11B shows the Kaplan-Meier curves and LogRank (Mantel-Cox) test (*comparing all groups vs. vehicle-treated controls; +comparing the PT+SIL+NR+FSL1-treated group vs. the PT+SIL+NR- or the PT+SIL+FSL1-treated group). Surprisingly, a synergistic effect was also found when NR and FLS1 were combined with PT and SIL, since the survival of the mice was increased for up to a year. Importantly, the combination of the four components provided protection and survival of up to 90% of the mice treated, showing that the combination of the four component achieves long term survival of irradiated mice.

The causes of death in γ irradiated mice (LD50/30) and the effect of the combined administration of the four compounds was studied, as shown in Figure 12. Swiss albino mice (n= 20/group) were treated with PT, SIL, NR and FSL-1 as scheduled previously and at the doses indicated above and the results showed that the full combination (PT+SII+NR+FSL-1) did avoid all deaths related to a) gastrointestinal and hematopoietic ARS, and b) nephropaty. The full combination also minimized deaths related to myeloid leukemia and infections/inflammations.

Next, the γ radiation induced toxicity and the prevention elicited by the combination of PT, SIL, NR and FSL-1 (abbreviated as PSNF) was studied. The results are shown in Table 3 below:

| | | **Control** | **γ-irradiated + vehicle** | **γ-irradiated + PSNF** |
|---|---|---|---|---|
| **Hematology** | | | | |
| | Hematocrit (%) | 39.8 ± 1.3 | 25.9 ± 1.0* | 33.1 ± 2.3*⁺ |
| | Hemoglobin (g/dL) | 12.4 ± 0.4 | 8.1 ± 0.3 | 10.1 ± 0.6*⁺ |
| | Erythrocytes (10⁶/µL) | 8.78 ± 0.19 | 6.0 ± 0.3* | 7.68 ± 0.15*⁺ |
| | Platelets (10³/µL) | 452 ± 27 | 135.8 ± 20.7* | 241 ± 21*⁺ |
| | Leukocytes (10³/µL) | 2.7 ± 0.2 | 0.84 ± 0.11* | 1.74 ± 0.11*⁺ |
| | Lymphocytes (10³/µL) | 1.42 ± 0.15 | 0.42 ± 0.08* | 0.96 ± 0.11*⁺ |
| | Neutrophiles (10³/µL) | 1.06 ± 0.05 | 0.34 ± 0.05* | 0.56 ± 0.09*⁺ |
| | Monocytes (10³/µL) | 0.09 ± 0.02 | 0.02 ± 0.03* | 0.07 ± 0.03⁺ |
| | Eosinophiles (10³/µL) | 0.09 ± 0.02 | 0.14 ± 0.05 | 0.09 ± 0.02 |
| | Basophiles (10³/µL) | 0 ± 0 | 0 ± 0 | 0 ± 0 |
| | Bone marrow cell count (10⁶ cells/femur) | 18.8 ± 1.0 | 7.4 ± 0.8* | 10.7 ± 0.9*⁺ |
| | Plasma osmolarity (mOsm/kg) | 270 ± 13 | 240 ± 11* | 265 ± 18⁺ |

| **Clinical chemistry** | | | | |
|---|---|---|---|---|
| | Urea (mg/dL) | 47.4 ± 1.3 | 56.5 ± 1.0* | 47 ± 3⁺ |
| | Uric acid (mg/dL) | 1.78 ± 0.08 | 1.28 ± 0.13* | 1.58 ± 0.08⁺ |
| | Total protein (g/dL) | 3.96 ± 0.11 | 3.20 ± 0.16* | 3.54 ± 0.05*⁺ |
| | Albumin (g/dL) | 3.06 ± 0.11 | 2.74 ± 0.09* | 3.06 ± 0.05 |
| | Creatinine (mg/dL) | 0.40 ± 0.07 | 0.48 ± 0.04 | 0.40 ± 0.07⁺ |
| | Glucose (mg/dL) | 135 ± 3 | 122 ± 4* | 131.4 ± 1.3⁺ |
| | Total bilirubin (mg/dL) | 0.52 ± 0.04 | 0.38 ± 0.04* | 0.48 ± 0.04⁺ |
| | Direct bilirubin (mg/dL) | 0.09 ± 0.02 | 0.06 ± 0.02 | 0.08 ± 0.03 |
| | Aspartate aminotransferase (IU/L) | 145 ± 10 | 264 ± 9* | 197 ± 16*⁺ |
| | Alanine aminotransferase (IU/L) | 7.1 ± 0.3 | 50 ± 4* | 28 ± 4*⁺ |
| | γ-glutamyl-transpeptidase (IU/L | 1.82 ± 0.13 | 3.5 ± 0.3* | 2.12 ± 0.19⁺ |
| | Alkaline phosphatase (IU/L) | 122 ± 7 | 163 ± 8* | 136 ± 12⁺ |
| | Lactate dehydrogenase (IU/L) | 197 ± 10 | 363 ± 14* | 246 ± 10*⁺ |
| | Sodium (mEq/L) | 143 ± 6 | 119 ± 5* | 138 ± 5⁺ |
| | Potassium (mEq/L) | 8.04 ± 0.11 | 10.6 ± 0.4* | 8.7 ± 0.4⁺ |
| | Chloride (mEq/L) | 95 ± 4 | 114 ± 5* | 100 ± 2⁺ |

| **Isolated CD2⁺ lymphocytes** | | | | |
|---|---|---|---|---|
| | GSH (nmol/10⁶ cells) | 5.5 ± 0.2 | 3.02 ± 0.19* | 4.40 ± 0.16*⁺ |
| | Cell volume (µm³) | 176 ± 5 | 196 ± 6* | 182 ± 4⁺ |
| | Glucose utilization (µmol/g x min) | 1.10 ± 0.07 | 0.60 ± 0.10* | 1.00 ± 0.07⁺ |
| | Glutamine utilization (µmol/g x min) | 3.32 ± 0.08 | 2.02 ± 0.13* | 2.98 ± 0.08*⁺ |

As can be seen in Table 3, treatment with the full combination (PT+SII+NR+FSL-1) helps to improve the radiation-induced toxicity.

Lastly, to elucidate if the treatment with the four combined compounds (PT + SIL + NR + FSL-1; PSNF) would interfere with the therapeutic efficacy of anti-cancer radiotherapy (X rays) in tumours xenografts, female nu/nu nude mice (ages 6-8 weeks) (n= 5 mice/group) were inoculated s.c. with 5 ×10⁶ A549 (lung adenocarcinoma) or MDA-MB-231 (triple negative mammary carcinoma) cells per mouse. Tumor volume was calculated based on two dimensions, measured using calipers, and was expressed in cubic millimeters according to the formula: V = 0.5a x b², where a and b are the long and short diameters of the tumor, respectively. Tumors were irradiated with X-rays (10 Gy) using a 6-keV SL75 linear accelerator from Philips. Single fraction radiotherapy was administered at a rate of 5.0 Gy/min. The protocol for PSNF administration was identical to that explained above, and the radiotherapy was administered on day 21. Statistical analyses were performed using Student's t-test (P< 0.01; *comparing all groups vs. controls; +comparing the γ rays+PSNF-treated group vs. the group treated with γ rays alone).

The results are shown in Figure 13, where can be seen that the combination of the four compounds does not diminish the efficacy of anti-cancer treatment with X rays, proving the feasibility of using these compounds in patients undergoing said radiotherapy treatment.

Altogether, these results support the use of compositions comprising at least PT and SIL, and optionally NR and FSL1, to protect and mitigate from the damage induced by radiation. It is important to note that the radioprotective of these compounds was extended for long period of times (1 year or longer) as shown in Figure 3A and Figure 11B.

### EXAMPLE 5: Pterostilbene, silibinin and nicotinamide riboside decrease cellular DNA damage, and FSL1 lipopeptide promotes recovery of the hematopoietic system.

As shown in Fig. 14a, NR treatment increases the NAD+ content in different cell types and also prevents the decrease of NAD+ induced by γ radiation. PT+SIL administered alone did not affect the NAD+ levels in any of the cells studied and did not affect the increase elicited by NR (results not shown). Administration of FK866, a specific noncompetitive inhibitor of nicotinamide phosphoribosyltransferase (NAMPT), decreased NAD+ levels in mice treated with PT+SIL+NR or PT+SIL+NR+ γ rays (Fig. 14a). FK866-induced NAD+ depletion associated with a drastic decrease in NAMPT activity (Fig. 14b) (PT, SIL, NR, FSL1 and/or γ radiation did not influence the rate of FK866-induced inhibition of the NAMPT activity, not shown). Importantly, as shown in Fig. 14c and based on the DNA damage COMET assay, PT+SIL or NR favored DNA recovery and their combination was the most effective (Fig. 14c). Presumably, the effect of the polyphenols is an antioxidant-related mechanism, whereas the NR-induced increase in NAD+ directly promotes the DNA repair activity. As shown in Fig. 14d, FSL-1 increases the hematopoietic colony forming cells CFU-GM (colony forming unit-granulocyte, macrophage) and CFU-GEMM (colony forming unit-granulocyte, erythroid, macrophage, megakaryocyte). Thus confirming the post-irradiation hematopoietic recovery suggested by the data under Hematology in Table 3 above.

### Nrf2, NF-κB, PGC-1a and PARP1 interactions in the radioprotective mechanism.

Isolated epithelial intestinal cells were used to investigate potential signaling pathways that may be involved in the protective effect elicited by the combination of PT+SIL. Based on the data reported in Figs. 5-8, PT+SIL exert protection against the γ radiation-induced oxidative damage. Presumably, the observed protection is due to induction of Nrf2 (nuclear factor erythroid 2-related factor 2)-dependent antioxidant defenses (Figs. 5-8). Anti-inflammatory properties have been reported for both polyphenols thus suggesting the involvement of NF-κB (nuclear factor kappa-light-chain-enhancer of activated B cells)-dependent signaling. NR-induced increase of NAD+ will also favor PARP1 (Poly [ADP-ribose] polymerase 1)-dependent DNA repair, sirtuin activity, and PGC-1α(peroxisome proliferator-activated receptor γ co-activator 1 α) activation. Moreover, PT may induce PGC-1α, Nrf2 activation, and Sirt1 expression; whereas SIL could influence Sirt3 expression and PARP1 activity. As shown in Fig. 15a, as compared to controls, both PT and SIL increased nuclear Nrf2 while decreasing nuclear NF-κB levels. In the case of Nrf2, the effect of PT is more pronounced than that exerted by SIL. These effects, as a whole, suggest a cooperative effect in promoting the antioxidant defenses while downregulating the NF-κB-dependent proinflammatory response. pPGC-1α was also increased by PT (Fig. 15a). Sirt1 levels were increased by PT. Interestingly, although SIL alone did not affect Sirt1 levels, the combination of the two polyphenols increased Sirt1 levels more than PT alone (Fig. 15a). Both, PT and SIL increased Sirt3 levels, although the effect of SIL on Sirt3 was more robust (Fig. 15a). Both PT and SIL increased PARP1 (Fig. 15a). Fig. 15b outlines the possible molecular interrelationships between these signaling cascades, and the steps where PT and SIL may exert their effects. In order to further investigate which specific molecules may be determinant in the radioprotective effect, we used isolated IECs (from control or PT+SIL-treated mice, as in Fig. 11a) and a gene silencing-based approach. As shown in Fig. 15c, siRNAs to target mouse Nfκbie, Nfe2I2, Ppargc1, Sirt1, Sirt3, Parp1, SOD2 or GPX1 gene expression facilitate the increase of free NF-κB and the downregulation of all the other molecular targets. Isolated IECs were cultured for 24 h in the absence or presence of PT+SIL at concentrations (see the caption of Fig. 15) that ensure preservation of the in vivo-induced radioprotective defense, and in the presence of specific siRNAs. As shown in Fig. 15d, PT and SIL drastically decreased the γ irradiation-induced cell death observed in control (vehicle-treated) cells. Cell death analysis, based on the effect of specific RNAs in cells subjected to γ rays, shows that Nrf2, pPGC-1α, SOD2 and Sirt3 are mainly responsible (Fig. 15d) for the radioprotective effect elicited by the treatment with polyphenols.

### CONCLUSIONS

Here, we investigated the effect of systemically administered PT against γ radiation, and found a high percentage of survival by combining PT and SIL. Moreover, the association of potential radioprotectors and radiomitigators as a strategy to decrease radiation-induced damage and enhance survival still is an underdeveloped field. The present work shows the potency of a combination capable of achieving long-term survival in mice subjected to lethal (LD50/30) γ irradiation. This novel strategy, which demonstrates prolonged survival, may serve as a paradigm moving forward. Thus, considering the large number of molecules being tested as radioprotectors and/or radiomitigators, this paradigm may help to open up the field. PT and SIL upregulate the antioxidant defenses in different cell types and decrease the γ radiation-induced oxidative damage and cytogenetic alterations (chromosomal aberrations and micronuclei formation). As shown in Fig. 15, the protection elicited by PT and SIL may involve different signaling mechanisms which are interrelated. Oxidative stress and inflammation are direct pathophysiological mechanisms activated by exposure of cells to harmful ionizing radiations. PT and SIL increase nuclear Nrf2 and the antioxidant response in different cell types (Fig. 15), whereas they decrease NF-□B (Fig. 15) and, presumably, the radiation-associated inflammatory response. In addition, PT increases Sirt1 levels, which facilitates deacetylation of PGC-1α (Fig. 15). PGC-1α is an inducer of Sirt3 expression, and as shown in Fig. 15, SIL treatment also associates with an increase in Sirt3 levels. Mitochondrial SOD2 (deacetylated by Sirt3) and GSH peroxidase (which depends on the import of GSH from the cytosol) would work to decrease mitochondria-derived ROS and prevent apoptosis activation. Importantly, as shown in Fig. 15c and 15d, a gene silencing-based approach identified the pathway linking Nrf2, Sirt3 and SOD2 as key for the radioprotective effect elicited by the combined treatment with PT and SIL. In addition, NR generates NAD+ to support Sirt1 and Sirt3 activities and the PARP1-dependent DNA repair (Fig. 15). PARP1 activity is also induced by both polyphenols (Fig. 15). NR, a pyridine-nucleoside form of vitamin B3, is a precursor to NAD+ with superior pharmacokinetic profile relative to other forms of B3 (nicotinic acid and nicotinamide), therefore it represents an optimum NAD+ booster.

This work demonstrates that a combination of two radioprotectors, pterostilbene and silibinin, with two radiomitigators, nicotinamide riboside and fibroblast-stimulating lipoprotein 1, can confer long-term protection of normal tissues against a lethal dose of radiation in mice. The established safety profiles of these four molecules clearly warrants continued research to translate this combination for its application in humans. Ideal radioprotectors or radiomitigators should be stable, offer the possibility of easy administration, have no relevant systemic toxicity, and protect normal tissues from radiation-induced damages. The proposed combination meets these criteria, while demonstrating high efficacy.

## Claims

1. A **composition** comprising a) Pterostilbene, or a salt thereof, and b) Silibinin, or a salt thereof.

2. The composition according to claim 1, wherein the composition further comprises at least one or more radiomitigator compound/s, preferably selected from
a. NAD+ booster, and/or
b. Fibroblast-Stimulating Lipopeptide-1,
or any salts thereof.

3. The composition according to any of claims 1 or 2, wherein the Pterostilbene is Pterostilbene phosphate disodium salt and/or wherein the Silibinin is silibinin-C-2',3-dihydrogen succinate, disodium salt.

4. The composition according to claim 3, wherein the NAD+ booster is Nicotinamide Riboside.

5. Pterostilbene, or a pharmaceutically acceptable salt thereof, **for use** in the prevention, amelioration or reduction of diseases induced by ionizing radiation in a subject, in combination with Silibinin, or a pharmaceutically acceptable salt thereof, wherein the administration is carried out before the radiation, or after the radiation, or during the radiation; and wherein the combination is herein understood as the simultaneous or sequential administration, in any order, of a) Pterostilbene, or a pharmaceutically acceptable salt thereof, and b) Silibinin, or a pharmaceutically acceptable salt thereof, to said subject.

6. Pterostilbene for use according to claim 5, wherein the use further comprises the administration in combination with at least one or more radiomitigator compound/s, wherein the combination is herein understood as the simultaneous or sequential administration of a) Pterostilbene, or a pharmaceutically acceptable salt thereof, b) Silibinin, or a pharmaceutically acceptable salt thereof, and c) at least one or more radiomitigator compound/s, or any pharmaceutically acceptable salt thereof, to said subject.

7. Pterostilbene for use according to claim 6, wherein the at least one or more radiomitigator compound/s is a NAD+ booster and/or a Fibroblast-Stimulating Lipopeptide, or any pharmaceutically acceptable salts thereof.

8. Pterostilbene for use according to any of claims 5 to 7, wherein the diseases induced by ionizing radiation are related to DNA damage, radiation-induced cytotoxicity, genotoxicity and/or oxidative cellular damage, preferably acute radiation syndrome and/or chronic radiation syndrome.

9. A **non-therapeutic cosmetic** use of Pterostilbene, or a salt thereof, in combination with Silibinin, or a salt thereof, wherein the combination is herein understood as the simultaneous or sequential administration, in any order, of a) Pterostilbene, or a salt thereof, and b) Silibinin, or a salt thereof, to said subject.

10. The non-therapeutic cosmetic use according to claim 9, wherein the use is to protect, ameliorate and/or reduce the adverse effects of the sun on human skin and lips, such as age spots, sunburn, sun spots, lines, fine lines, wrinkles, crow's feet, spider veins, stretch marks, dark eye circles, hyperpigmentation, hypopigmentation, discoloration, uneven skin tone, dullness, freckles, skin breakout, blemishes, skin fragility, dryness, patchiness, tactile roughness, chapping, sagginess, thinning, enlarged pores.

11. Pterostilbene, or a salt thereof, in combination with Silibinin, or a salt thereof, **for use to increase the intracellular levels of Sirtuins** in a patient in need thereof, wherein said increase is as compared to the intracellular levels of Sirtuins in the same patient before treatment with Pterostilbene, or a salt thereof, in combination with Silibinin, or a salt thereof.

12. Pterostilbene, or a salt thereof, in combination with Silibinin, or a salt thereof, for use according to claim 11, wherein the patient in need thereof is a patient suffering from a neurodegenerative, cardiovascular, pulmonary, metabolic, or aged-related disorders; preferably wherein the disorder is selected from the list consisting of Alzheimer or other dementias or cognitive impairment associated to aging, Parkinson, Huntington disease, Multiple sclerosis, Kennedy disease (Spinal and bulbar muscular atrophy), kidney disease, cerebrovascular disease, hypertension, Chronic obstructive pulmonary disease, Amyotrophic lateral sclerosis and Diabetes.

13. Pterostilbene, or a salt thereof, in combination with Silibinin, or a salt thereof, **for use as a nutraceutical composition,** wherein the combination is herein understood as the simultaneous or sequential administration, in any order, of a) Pterostilbene, or a salt thereof, and b) Silibinin, or a salt thereof, to said subject.

14. Pterostilbene, or a salt thereof, in combination with Silibinin, or a salt thereof, for use according to claim 13, wherein the use as a nutraceutical composition is for the treatment or prevention of diseases induced by ionizing radiation, preferably related to DNA damage, radiation-induced cytotoxicity, genotoxicity and/or oxidative cellular damage; for the treatment or prevention of neurodegenerative, cardiovascular, pulmonary, metabolic, or aged-related disorders, and/or for the protection or repairment of the adverse effects of the sun on human skin or lips, .

15. A **kit of parts** comprising at least two recipients comprising at least Pterostilbene, or a salt thereof, and Silibinin, or a salt thereof, and optionally at least one or more radiomitigator compound/s.

## Patentansprüche

1. Zusammensetzung umfassend a) Pterostilbene, oder ein Salz davon, und b) Silibinin, oder ein Salz davon.

2. Zusammensetzung nach Anspruch 1, wobei die Zusammensetzung zusätzlich mindestens eine oder mehrere strahlungsmindernde Verbindungen umfasst, vorzugsweise ausgewählt aus
a. NAD+ Booster, und/oder
b. fibroblastenstimulierendem Lipopeptid-1,
oder jede Salz davon.

3. Zusammensetzung nach einem der Ansprüche 1 bis 2, wobei das Pterostilbene Pterostilbenephosphat-Dinatriumsalz ist und/oder wobei das Silibinin Silibinin-C-2',3-dihydrogensuccinat-Dinatriumsalz ist.

4. Zusammensetzung nach Anspruch 3, wobei der NAD+ Booster Nicotinamidribosid ist.

5. Pterostilbene, oder ein pharmazeutisch akzeptables Salz davon, für dessen Verwendung bei der Vorbeugung, Verbesserung oder Verminderung von durch ionisierende Strahlung induzierten Krankheiten in einem Individuum, in Kombination mit Silibinin, oder einem pharmazeutisch akzeptablen Salz davon, wobei die Verabreichung vor der Strahlung, oder nach der Strahlung oder während der Strahlung durchgeführt wird; und wobei die Kombination hier als die gleichzeitige oder sequenzielle Verabreichung, in jeder Reihenfolge, von a) Pterostilbene, oder einem pharmazeutisch akzeptablen Salz davon, und b) Silibinin, oder einem pharmazeutisch akzeptablen Salz davon, zum genannten Individuum verstanden wird.

6. Pterostilbene für dessen Verwendung nach Anspruch 5, wobei die Verwendung zusätzlich die Verabreichung in Kombination mit mindestens einer oder mehreren strahlungsmindernden Verbindungen umfasst, wobei die Kombination hier als die gleichzeitige oder sequenzielle Verabreichung von a) Pterostilbene, oder einem pharmazeutisch akzeptablen Salz davon, b) Silibinin, oder einem pharmazeutisch akzeptablen Salz davon, und c) mindestens einer oder mehreren strahlungsmindernde Verbindungen, oder jedem pharmazeutisch akzeptablen Salz davon, zum genannten Individuum verstanden wird.

7. Pterostilbene für dessen Verwendung nach Anspruch 6, wobei die mindestens eine oder mehreren strahlungsmindernden Verbindungen ein NAD+ Booster und/oder ein fibroblastenstimulierendes Lipopeptid, oder jedes pharmazeutisch akzeptable Salz davon ist.

8. Pterostilbene für dessen Verwendung nach einem der Ansprüche 5 bis 7, wobei die durch ionisierende Strahlung induzierten Krankheiten mit DNA-Schaden, strahlungsinduzierter Zytotoxizität, Genotoxizität und/oder oxidativer Zellschädigung, vorzugsweise akutem Strahlensyndrom und/oder chronischem Strahlensyndrom, verbunden sind.

9. Nicht-therapeutische kosmetische Verwendung von Pterostilbene, oder einem Salz davon, in Kombination mit Silibinin, oder einem Salz davon, wobei die Kombination hier als die gleichzeitige oder sequenzielle Verabreichung, in jeder Reihenfolge, von a) Pterostilbene, oder einem Salz davon, und b) Silibinin, oder einem Salz davon, zum genannten Individuum verstanden wird.

10. Nicht-therapeutische kosmetische Verwendung nach Anspruch 9, wobei die Verwendung für den Schutz, die Verbesserung und/oder die Verminderung der Nebenwirkungen der Sonne auf der menschlichen Haut und den Lippen, wie Altersflecke, Sonnenbrand, Sonnenflecke, Linien, feiner Linien, Falten, Krähenfüße, Besenreiser, Dehnungsstreifen, dunkler Augenringe, Hyperpigmentierung, Hypopigmentierung, Verfärbung, ungleichmäßigen Hautton, Glanzlosigkeit, Sommersprossen, Hautausbruch, Hautunreinheiten, Brüchigkeit der Haut, Trockenheit, Ungleichmäßigkeit, taktiler Rauheit, Rissigkeit, Schlaffheit, Verdünnung, erweiterter Poren ist.

11. Pterostilbene, oder ein Salz davon, in Kombination mit Silibinin, oder einem Salz davon, für dessen Verwendung zur Erhöhung der intrazellulären Spiegel von Sirtuinen in einem Patienten, welcher es benötigt, wobei die genannte Erhöhung im Vergleich zu den intrazellulären Spiegeln von Sirtuinen im gleichen Patienten vor der Behandlung mit Pterostilbene, oder einem Salz davon, in Kombination mit Silibinin, oder einem Salz davon, ist.

12. Pterostilbene, oder ein Salz davon, in Kombination mit Silibinin, oder einem Salz davon, für dessen Verwendung nach Anspruch 11, wobei der Patient, welcher es benötigt, ein Patient ist, welcher unter einer neurodegenerativen, kardiovaskulären, pulmonalen, metabolischen oder altersbedingten Krankheit leidet; wobei vorzugsweise die Krankheit aus der Liste bestehend aus Alzheimer oder anderen Demenzen oder einer mit dem Alter verbundenen kognitiven Störung, Parkinson, Huntington-Krankheit, multipler Sklerose, Kennedy-Krankheit (Bulbo-spinaler Muskelatrophie), Nierenerkrankung, Hirngefäßkrankheit, Bluthochdruck, chronisch obstruktiver Lungenerkrankung, amyotropher Lateralsklerose und Diabetes ausgewählt wird.

13. Pterostilbene, oder ein Salz davon, in Kombination mit Silibinin, oder einem Salz davon, für dessen Verwendung als nutrazeutische Zusammensetzung, wobei die Kombination hier als die gleichzeitige oder sequenzielle Verabreichung, in jeder Reihenfolge, von a) Pterostilbene, oder einem Salz davon, und b) Silibinin, oder einem Salz davon, zum genannten Individuum verstanden wird.

14. Pterostilbene, oder ein Salz davon, in Kombination mit Silibinin, oder einem Salz davon, für dessen Verwendung nach Anspruch 13, wobei die Verwendung als nutrazeutische Zusammensetzung für die Behandlung oder Vorbeugung von durch ionisierende Strahlung induzierten Krankheiten, vorzugsweise mit DNA-Schaden, strahlungsinduzierter Zytotoxizität, Genotoxizität und/oder oxidativer Zellschädigung verbunden; für die Behandlung oder Vorbeugung von neurodegenerativen, kardiovaskulären, pulmonalen, metabolischen oder altersbedingten Krankheiten, und/oder für den Schutz oder die Behebung von Nebenwirkungen der Sonne auf der menschlichen Haut oder den Lippen ist.

15. Teilesatz umfassend mindestens zwei Behälter, welche mindestens Pterostilbene, oder ein Salz davon, und Silibinin, oder ein Salz davon, und wahlweise mindestens eine oder mehrere strahlungsmindernde Verbindungen umfassen.

## Revendications

1. Composition comprenant a) du ptérostilbène, ou un sel de celui-ci, et b) de la silibinine, ou un sel de celle-ci.

2. Composition selon la revendication 1, dans laquelle la composition comprend en outre au moins un ou plusieurs composés radio-atténuateurs, de préférence choisis parmi
a. un renforçateur de NAD+, et/ou
b. lipopeptide stimulant des fibroblastes 1,
ou un sel quelconque de ceux-ci.

3. Composition selon l'une quelconque des revendications 1 ou 2, dans laquelle le ptérostilbène est le sel disodique de phosphate de ptérostilbène et/ou dans laquelle la silibinine est le sel disodique de silibinine-C-2',3-dihydrogénosuccinate.

4. Composition selon la revendication 3, dans laquelle le renforçateur de NAD+ est le nicotinamide riboside.

5. Ptérostilbène, ou un sel pharmacéutiquement acceptable de celui-ci, pour son utilisation dans la prévention, l'amélioration ou la réduction de maladies induites par les rayonnements ionisants chez un sujet, en combinaison avec la silibinine, ou un sel pharmacéutiquement acceptable de celle-ci, dans lequel l'administration est réalisée avant les rayonnements, ou après les rayonnements, ou pendant les rayonnements; et dans lequel la combinaison est ici comprise comme l'administration simultanée ou séquentielle, dans n'importe quel ordre, de a) ptérostilbène, ou un sel pharmacéutiquement acceptable de celui-ci, et b) silibinine, ou un sel pharmacéutiquement acceptable de celle-ci, audit sujet.

6. Ptérostilbène pour son utilisation selon la revendication 5, dans lequel l'utilisation comprend en outre l'administration en combinaison avec au moins un ou plusieurs composés radio-atténuateurs, dans lequel la combinaison est ici comprise comme l'administration simultanée ou séquentielle de a) ptérostilbène, ou un sel pharmacéutiquement acceptable de celui-ci, b) silibinine, ou un sel pharmacéutiquement acceptable de celle-ci, et e) au moins un ou plusieurs composés radio-atténuateurs, ou un sel pharmacéutiquement acceptable quelconque de ceux-ci, audit sujet.

7. Ptérostilbène pour son utilisation selon la revendication 6, dans lequel l'au moins un ou plusieurs composés radio-atténuateurs sont un renforçateur de NAD+ et/ou un lipopeptide stimulant des fibroblastes, ou un sel pharmacéutiquement acceptable quelconque de ceux-ci.

8. Ptérostilbène pour son utilisation selon l'une quelconque des revendications 5 à 7, dans lequel les maladies induites par les rayonnements ionisants sont liées à des dommages de l'ADN, cytotoxicité induite par les rayonnements, génotoxicité et/ou dommage cellulaire oxydatif, de préférence le syndrome d'irradiation aigu et/ou le syndrome d'irradiation chronique.

9. Utilisation cosmétique non-thérapeutique du ptérostilbène, ou un sel de celui-ci, en combinaison avec de la silibinine, ou un sel de celle-ci, dans laquelle la combinaison est ici comprise comme l'administration simultanée ou séquentielle, dans n'importe quel ordre, de a) ptérostilbène, ou un sel de celui-ci, et b) silibinine, ou un sel de celle-ci, audit sujet.

10. Utilisation cosmétique non-thérapeutique selon la revendication 9, dans laquelle l'utilisation est pour protéger, améliorer et/ou réduire les effets indésirables du soleil sur la peau et les lèvres humains, tels que les taches de vieillesse, les coups de soleil, les taches solaires, les lignes, les ridules, les rides, les pattes d'oie, les veines d'araignée, les stries, les cernes, l'hyperpigmentation, l'hypopigmentation, la décoloration, le teint irrégulier, le teint terne, les taches de rousseur, les éruptions cutanées, les imperfections, la fragilité cutanée, la sécheresse, les taches, la rugosité tactile, les gerçures, le relâchement, l'amincissement, les pores dilatés.

11. Ptérostilbène, ou un sel de celui-ci, en combinaison avec de la silibinine, ou un sel de celle-ci, pour son utilisation pour augmenter les niveaux intracellulaires de sirtuines chez un patient en ayant besoin, dans lequel ladite augmentation est par rapport aux niveaux intracellulaires de sirtuines chez le même patient avant le traitement avec du ptérostilbène, ou un sel de celui-ci, en combinaison avec de la silibinine, ou un sel de celle-ci.

12. Ptérostilbène, ou un sel de celui-ci, en combinaison avec de la silibinine, ou un sel de celle-ci, pour son utilisation selon la revendication 11, dans lequel le patient en ayant besoin est un patient souffrant d'un trouble neurodégénératif, cardiovasculaire, pulmonaire, métabolique, ou lié à l'âge; de préférence dans lequel le trouble est choisi parmi la liste constituée de la maladie d'Alzheimer ou d'autres démences ou trouble cognitif lié à l'âge, la maladie de Parkinson, la maladie de Huntington, la sclérose multiple, la maladie de Kennedy (atrophie musculaire spinale et bulbaire), une maladie rénale, une maladie cérébrovasculaire, l'hypertension, la maladie pulmonaire obstructive chronique, la sclérose latérale amyotrophique et le diabète.

13. Ptérostilbène, ou un sel de celui-ci, en combinaison avec de la silibinine, ou un sel de celle-ci, pour son utilisation comme composition nutraceutique, dans lequel la combinaison est ici comprise comme l'administration simultanée ou séquentielle, dans n'importe quel ordre, de a) ptérostilbène, ou un sel de celui-ci, et b) silibinine, ou un sel de celle-ci, audit sujet.

14. Ptérostilbène, ou un sel de celui-ci, en combinaison avec de la silibinine, ou un sel de celle-ci, pour son utilisation selon la revendication 13, dans lequel l'utilisation comme composition nutraceutique est pour le traitement ou la prévention de maladies induites par les rayonnements ionisants, de préférence liées à des dommages de l'ADN, cytotoxicité induite par les rayonnements, génotoxicité et/ou dommage cellulaire oxydatif, pour le traitement ou la prévention de troubles neurodégénératifs, cardiovasculaires, pulmonaires, métaboliques, ou liés à l'âge, et/ou pour la protection ou la réparation des effets indésirables du soleil sur la peau ou les lèvres humains.

15. Kit de pièces comprenant au moins deux récipients comprenant au moins du ptérostilbène, ou un sel de celui-ci, et de la silibinine, ou un sel de celle-ci, et optionnellement au moins un ou plusieurs composés radio-atténuateurs.
